# EUROPEAN PATENT APPLICATION

(11) **EP 3 723 040 A1**
(43) Date of publication of application: **14.10.2020**
(21) Application number: 19885077.8
(22) Date of filing: 05.11.2019
(51) Int. Cl.: G06T 7/00

(54) **INFORMATION PROCESSING METHOD AND INFORMATION PROCESSING SYSTEM**

(30) Priority: 12.11.2018 JP 2018212136; 25.10.2019 JP 2019194798
(71) Applicant: Sony Corporation, 108-0075 Tokyo (JP)
(72) Inventor: WATANABE, Shinji, Tokyo 108-0075 (JP); YAMANE, Kenji, Tokyo 108-0075 (JP)
(74) Representative: D Young & Co LLP
(86) International application number: PCT/JP2019/043338
(87) International publication number: WO 2020/100660

(57) **Abstract**

An information processing method including: learning, based on a first specimen image of a first specimen to which a first effect has been applied, an evaluator parameter of an evaluator that performs evaluation of the first specimen, the first specimen image having been captured by a first imaging device; storing, into a storage medium, evaluation setting information including the evaluator parameter and learning environment information indicating a learning environment for the evaluator parameter; acquiring the evaluation setting information stored in the storage medium; and reproducing, based on the evaluation setting information acquired, an environment equivalent to the learning environment, the environment serving as an evaluation environment for evaluation of a second specimen of which an image is captured by a second imaging device different from the first imaging device.

## Description

### Field

The present disclosure relates to information processing methods and information processing systems.

### Background

Evaluation of specimens has been widely been conducted recently for the purpose of treatment, research, and the like, the evaluation being achieved by: sampling of a specimen, such as cells or blood, from an organism, such as a human; subsequent application of an effect, such as staining, to the specimen; and observation of a specimen image captured by a microscope thereafter. There is a demand for a technique for more appropriate evaluation of specimens based on specimen images.

For example, in Patent Literature 1 cited below, a technique for correction of a specimen image has been disclosed, the correction being performed such that a pigment quantity distribution of the specimen image approximates to the quantity of the pigment in a standard specimen image, for the purpose of standardization of variation in staining by image processing, the variation being caused when a biological tissue is stained for observation.

### Citation List

### Patent Literature

Patent Literature 1: JP 2009-14355 A

### Summary

### Technical Problem

Mechanical evaluation by artificial intelligence has been attempted recently for facilitation of evaluation of specimens based on specimen images. Learning by AI is often performed with training data that are a large number of specimen images having various parameters in common, the various parameters including an effect parameter and a microscope parameter. When AI that has finished learning is used, an appropriate evaluation result is able to be acquired by evaluation of a specimen image acquired by use of parameters that are the same as those in the learning.

The above condition is easily satisfied, when a person who performs learning in AI is identical to a person who uses the AI, for example, when, in a specific hospital: learning in AI is performed with specimen images of a specific organ, the specimen images having been captured with specific parameters by a specific microscope; and the AI is used. However, when a user who is different from the person who has performed the learning uses the AI, the above condition is not easily satisfied. Therefore, a scheme is desirably provided, the scheme being for: facilitation of satisfaction of the above condition even if a user different from the person who has performed the learning uses the AI; or facilitation of acquisition of an appropriate evaluation result by use of AI even if the above condition is not satisfied.

Accordingly, the present disclosure provides a scheme for facilitation of evaluation of specimens based on specimen images, the evaluation being performed by use of AI.

### Solution to Problem

According to the present disclosure, an information processing method is provided that includes: learning, based on a first specimen image of a first specimen to which a first effect has been applied, an evaluator parameter of an evaluator that performs evaluation of the first specimen, the first specimen image having been captured by a first imaging device; storing, into a storage medium, evaluation setting information including the evaluator parameter and learning environment information indicating a learning environment for the evaluator parameter; acquiring the evaluation setting information that has been stored in the storage medium; and reproducing, based on the evaluation setting information acquired, an environment equivalent to the learning environment, the environment serving as an evaluation environment for evaluation of a second specimen of which an image is captured by a second imaging device different from the first imaging device.

Moreover, according to the present disclosure, an information processing method is provided that includes: learning, based on a first specimen image of a first specimen to which a first effect has been applied, an evaluator parameter of an evaluator that performs evaluation of the first specimen, the first specimen image having been captured by a first imaging device; and storing, into a storage medium, evaluation setting information including the evaluator parameter and learning environment information indicating a learning environment for the evaluator parameter.

Moreover, according to the present disclosure, an information processing method is provided that includes: acquiring, from a storage medium, evaluation setting information including: an evaluator parameter of an evaluator that performs evaluation of a first specimen to which a first effect has been applied, the evaluator parameter having been learnt based on a first specimen image of the first specimen, the first specimen image having been captured by a first imaging device; and learning environment information indicating a learning environment for the evaluator parameter; and reproducing, based on the evaluation setting information acquired, an environment equivalent to the learning environment, the environment serving as an evaluation environment for evaluation of a second specimen of which an image is captured by a second imaging device different from the first imaging device.

### Brief Description of Drawings

FIG. 1 is a diagram illustrating an example of a configuration of a diagnostic system according to an embodiment of the present disclosure.
FIG. 2 is a diagram illustrating an example of a functional configuration of the diagnostic system according to the embodiment.
FIG. 3 is a diagram illustrating flows of information related to generation of an evaluation recipe according to the embodiment.
FIG. 4 is a diagram illustrating an example of a UI according to the embodiment.
FIG. 5 is a flow chart illustrating an example of a flow of uploading processing for an evaluation recipe, the uploading processing being executed in a hospital server according to the embodiment.
FIG. 6 is a flow chart illustrating an example of a flow of storage processing for an evaluation recipe, the storage processing being executed in an evaluation recipe server according to the embodiment.
FIG. 7 is a flow chart illustrating an example of a flow of first reproduction processing executed in a hospital server and a terminal apparatus according to the embodiment.
FIG. 8 is a flow chart illustrating an example of a flow of second reproduction processing executed in the hospital server and the terminal apparatus according to the embodiment.
FIG. 9 is a diagram illustrating an example of information processing by use of evaluators according to the embodiment.
FIG. 10 is a diagram illustrating an example of the information processing by use of the evaluators according to the embodiment.
FIG. 11 is a diagram illustrating an example of information processing by use of the evaluators according to the embodiment, when correction is performed upon evaluation.
FIG. 12 is a diagram illustrating an example of information processing by use of the evaluators according to the embodiment, when correction is performed upon learning.
FIG. 13 is a diagram illustrating an example of a flow of reproduction processing executed by a generating unit and a reproducing unit, according to the embodiment.
FIG. 14 is a diagram illustrating an example of the flow of reproduction processing executed by the generating unit and the reproducing unit, according to the embodiment.
FIG. 15 is a block diagram illustrating an example of a hardware configuration of an information processing apparatus according to the embodiment.

### Description of Embodiments

Preferred embodiments of the present disclosure will hereinafter be described in detail, while reference is made to the appended drawings. Redundant explanation will be omitted by assignment of the same reference sign to components having substantially the same functional configuration, throughout this specification and the drawings.

Description will be made in the following order.
1. Introduction
2. Configuration Example
3. Details of Reproduction Processing
4. Example of UI
5. Flow of Processing
6. Application Examples
7. Other Embodiments
   7.1. Modified Examples of Evaluation Recipe
   7.2. Types of Correction
   7.3. Outline of Correction Processing
   7.4. Types of Correction Processing
   7.5. Use of Evaluation Recipe
   7.5.1. Information Processing Based on Correction upon Learning
   7.5.2. Information Processing Based on Correction upon Evaluation
   7.6. Modified Examples of Specimen Attribute Information
   7.7. Generation of Combined Recipe
   7.8. Modified Examples of Configuration
   7.9. Notation for User
   7.10. Notation for Medical Image
8. Example of Hardware Configuration
9. Conclusion

### 1. Introduction

In pathological diagnosis, parts cut out from organs serve as specimens. An effect in pathological diagnosis refers to staining cells according to a purpose upon sampling. For example, if evaluation of morphology is the purpose, hematoxylin-eosin (HE) staining is adopted, and if evaluation of tumor immunity is the purpose, immunohistochemistry (IHC) staining is adopted. For example, in evaluation of breast cancer, IHC staining, in which an HER2 protein, an ER protein, a PgR protein, or a Ki-67 protein is stained, is performed.

A specimen to which the effect has been applied is set on a stage of a digital microscope, and images of the specimen are consecutively captured while the imaging range is changed. The images consecutively captured are joined together and a single large specimen image (also called a pathological image) is thereby generated. This specimen image is also called whole slide imaging (WSI).

A technique is rapidly becoming widespread recently, the technique being where learning is performed in AI for support of pathological diagnosis based on WSI or a partial image cut out from the WSI (the WSI and the partial image both being also referred to generally as specimen images hereinafter) and the AI is used. In this learning in the AI, for example, training data are used, the training data having: specimen images serving as data; and information indicating tumor regions in the specimen images (the information also being referred to as annotation information), the information serving as labels. In this case, when a specimen image is input to the AI that has finished the learning, annotation information indicating a tumor region in the specimen image is output. AI that supports pathological diagnosis may be, instead of the AI for the detection of tumor regions described above: AI for classification of tumors into classes (for example, cancer grading); AI for cancer diagnosis for cancer/non-cancer determination; or AI for treatment prediction.

For improvement of accuracy of diagnosis by AI, various parameters including an effect parameter and a microscope parameter at the time of learning are desirably made the same as those at the time of diagnosis. For example, specimen images of the same specimen may look differently depending on characteristics of microscopes. Or, even if the parameters are not the same, image processing that compensates for the difference between the parameters is desirably applied to the specimen image. In any of these cases, accuracy of diagnoses by use of AI is able to be improved.

However, reproducing the same parameters at the time of diagnosis or compensating for the difference between the parameters has been difficult conventionally because the parameters for the learning are not retained. Therefore, the present disclosure provides a scheme for performing processing for: retaining parameters for learning; and reproducing the parameters upon diagnosis, or compensating for a difference between parameters.

### 2. Example of Configuration

### 2.1. Example of System Configuration

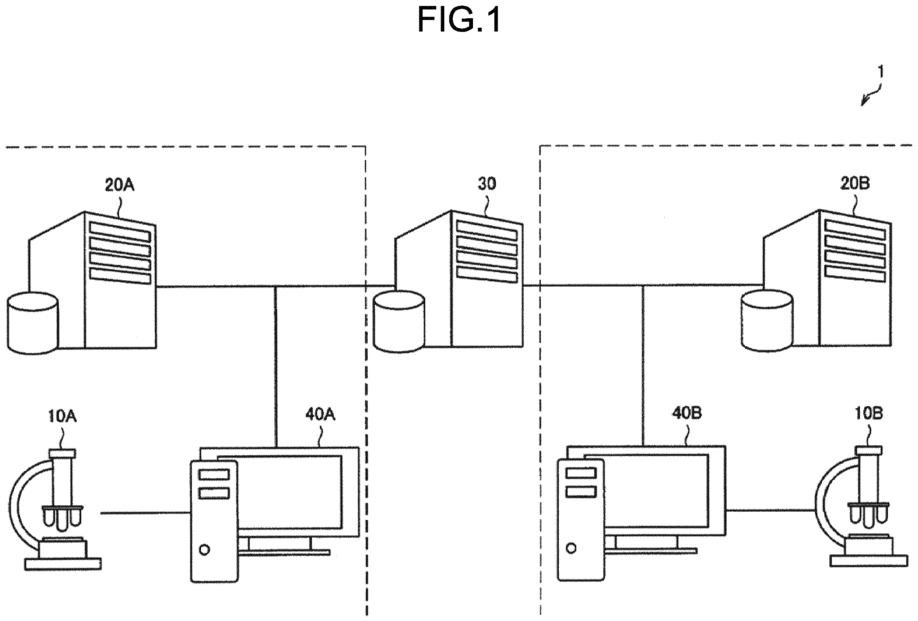

FIG. 1 is a diagram illustrating an example of a configuration of a diagnostic system according to an embodiment of the present disclosure. A diagnostic system 1 illustrated in FIG. 1 includes an imaging device 10 (10A and 10B), a hospital server 20 (20A and 20B), an evaluation recipe server 30, and a terminal apparatus 40 (40A and 40B).

### (1) Device Configurations

### Imaging Device 10

The imaging device 10 is a device that generates a specimen image by capturing an image of a specimen. The imaging device 10 is, for example, an electronic microscope having an imaging element attached to the microscope. The imaging device 10 generates a specimen image and outputs the generated specimen image, to the terminal apparatus 40.

### Hospital Server 20

The hospital server 20 is an information processing apparatus that manages various types of information related to diagnostic services in a hospital. In particular, the hospital server 20 performs generation and uploading of an evaluation recipe, or downloading of an evaluation recipe. Details of an evaluation recipe will be described later. For example, the hospital server 20 generates an evaluation recipe based on specimen images generated by the imaging device 10, and transmits the evaluation recipe to the evaluation recipe server 30. Furthermore, the hospital server 20 acquires, from the evaluation recipe server 30, an evaluation recipe for evaluation of a specimen image generated by the imaging device 10, and outputs the evaluation recipe, to the terminal apparatus 40.

### Evaluation Recipe Server 30

The evaluation recipe server 30 is an information processing apparatus that manages evaluation recipes. The evaluation recipe server 30 stores therein evaluation recipes received from the hospital server 20. Furthermore, the evaluation recipe server 30 transmits an evaluation recipe requested from the hospital server 20, to the hospital server 20.

### Terminal Apparatus 40

The terminal apparatus 40 is an information processing apparatus that performs evaluation based on a specimen image generated by the imaging device 10. The terminal apparatus 40 includes a user interface, and performs input of information through an employee of a hospital and output of information to an employee of the hospital.

### (2) Processing in Hospitals

### First Hospital

The imaging device 10A (corresponding to a first imaging device), the hospital server 20A, and the terminal apparatus 40A are located in a first hospital. The first hospital is a hospital where an evaluation recipe is generated.

The imaging device 10A generates a first specimen image of a first specimen to which a first effect has been applied, and outputs the first specimen image, to the terminal apparatus 40A. At the terminal apparatus 40A, evaluation based on the first specimen image is performed. For example, a result of evaluation by an employee of the first hospital is input to the terminal apparatus 40A. The hospital server 20A stores the first specimen image and meta-information of the first specimen image in association with each other, and generates an evaluation recipe based on information that has been stored. The hospital server 20A generates the evaluation recipe based on first specimen images having at least parts of their meta-information in common. Details of the meta-information will be described later.

An evaluation recipe is evaluation setting information indicating settings related to evaluation of a specimen image by use of an evaluator. Specifically, an evaluation recipe includes an evaluator parameter, and learning environment information indicating a learning environment for the evaluator parameter. The evaluator parameter is a parameter defining an evaluator that outputs, based on input information, evaluation result information. For example, at least a specimen image is input to an evaluator, and the evaluator outputs a result of evaluation of the specimen image (for example, a result of pathological diagnosis). An evaluator is formed of arbitrary AI, such as, for example, a neural network or a support vector machine (SVM). If an evaluator is formed of a neural network, the evaluator parameter is a set of weights indicating strengths of connections between nodes forming the neural network. A learning environment is characteristics common to specimen images used in learning of an evaluator parameter. Learning environment information is meta-information common to specimen images used in learning of an evaluator parameter.

An evaluation recipe is generated per learning environment. That is, an evaluator parameter is learnt per learning environment. For example, it is assumed that an evaluator parameter is learnt by collection of first specimen images of first specimens, which have been sampled from livers of males, and to which HE staining has been applied, for plural patients. In this case, an evaluation recipe is generated, the evaluation recipe including: the learnt evaluator parameter; learning environment information indicating "liver" and "HE staining"; and accompanying information on sex or the like.

The hospital server 20A transmits an evaluation recipe generated, to the evaluation recipe server 30 to store the evaluation recipe in the evaluation recipe server 30.

### Second Hospital

The hospital server 20B (corresponding to a second imaging device), the hospital server 20B, and the terminal apparatus 40B are located in a second hospital. The second hospital is a hospital where an evaluation recipe is used.

The hospital server 20B acquires an evaluation recipe from the evaluation recipe server 30, and outputs the evaluation recipe to the terminal apparatus 40B. The imaging device 10B generates a second specimen image of a second specimen to which a second effect has been applied, and outputs the second specimen image to the terminal apparatus 40B. The imaging device 10B may generate the second specimen image based on the evaluation recipe acquired from the evaluation recipe server 30. The terminal apparatus 40B performs evaluation of the second specimen image generated by the imaging device 10B by using the evaluation recipe acquired from the hospital server 20B.

At the second hospital, an evaluation recipe with a learning environment equivalent to an evaluation environment is used. An evaluation environment is an environment in which an evaluator parameter is used. Specifically, an evaluation environment is characteristics of meta-information of a second specimen image input to an evaluator to which an evaluator parameter has been applied.

For example, at the second hospital, when a second specimen image of a second specimen, which has been sampled from a liver of a male, and to which HE staining has been applied, is evaluated, an evaluation recipe having "liver" and "HE staining" as learning environment information is downloaded from the evaluation recipe server 30 and used. As a result, the learning environment and the evaluation environment thereby become equivalent to each other, and evaluation accuracy for the second specimen by use of an evaluator is thus able to be improved. Accompanying information on sex or the like, like "male",
is able to be presented as statistical information, like similarity of evaluation or the number of patients.

### (3) Description of Various Types of Information

### (3.1) Meta-information

Meta-information of a specimen image includes at least one of an image parameter, effect information, specimen attribute information, and evaluation result information.

### Image Parameter

An image parameter is a parameter related to generation of a specimen image. An image parameter includes an imaging parameter and an image processing parameter.

### Imaging Parameter

An imaging parameter is a parameter related to imaging of a specimen image by the imaging device 10. An imaging parameter may include at least one of identification information of the imaging device 10 (for example, a model number indicating a type of the imaging device 10), an automatic focusing setting, a magnification, an exposure time period, and a gamma correction value.

### Image Processing Parameter

An image processing parameter is information indicating image processing that has been applied to a specimen image captured by the imaging device 10. The image processing may include at least one of color correction or scaling. Furthermore, the image processing may include rotation or luminance correction.

### Effect Information

Effect information is a parameter related to an effect that has been applied to a specimen captured in a specimen image. In pathological diagnosis, effect information includes information on staining performed on a specimen. Effect information may include staining information indicating a type of staining, such as HE staining or IHC staining, and antibody information indicating a type of an antibody used in staining of HER2, ER, PgR, Ki-67, or the like. Furthermore, effect information may include information indicating a combination of a staining type and an antibody type, like IHC-HER2, IHC-ER, IHC-PgR, and IHC-Ki-67. In addition, drug administration and/or light stimulation may be applied as an effect or effects, and effect information may include information related to the effect/effects.

### Specimen Attribute Information

Specimen attribute information is information indicating an attribute of a specimen captured in a specimen image. Specimen attribute information includes information indicating what kind of specimen (that is, indicating whether a specimen is a biopsy specimen or a surgical specimen) of which organ the specimen is, and information related to a sampling source of the specimen. Specifically, specimen attribute information may include: the age, the sex, and the age at the time of examination, of the patient; the type of organ of the sampling source of a specimen; the sampling method; the examination date; a result of pathological diagnosis; pathological findings; a thumbnail image of the specimen; and genome information of the specimen. Furthermore, specimen attribute information may include the shape, morphology, and area of cells included in a specimen. These pieces of specimen attribute information may be used, not only for diagnostic support, but also for treatment support. For example, specimen attribute information may be used for treatment effect prediction for drugs, chemotherapy, or radiotherapy. In particular, genome information is one of important pieces of information that may be used for, not only diagnostic support, but also treatment support. For example, genome information is important for provision of medical care for individual patients (for example, companion diagnostics).

### Evaluation Result Information

Evaluation result information is information indicating a result of evaluation based on a specimen image. In pathological diagnosis, evaluation result information is a result of pathological diagnosis (that is, a definitive diagnosis). For example, evaluation result information includes annotation information indicating a tumor region, a result of caner/non-cancer determination, and a result of classification of a tumor into a class. Furthermore, evaluation result information may include the shape, morphology, and area, of a tumor region.

Hereinbefore, examples of pieces of information included in meta-information have been described. Information related to a first specimen image may be referred to with the word "first", and information related to a second specimen image may be referred to with the word "second". For example, an image parameter of a first specimen image may be referred to as a first image parameter, and specimen attribute information of a second specimen image may be referred to as second specimen attribute information.

### (3.2) Learning Environment Information

Learning environment information is information indicating a learning environment for an evaluator parameter. As described above, an evaluation recipe includes an evaluator parameter and learning environment information indicating a learning environment for the evaluator parameter. Learning environment information includes meta-information common to first specimen images used in learning of an evaluator parameter. For example, learning environment information includes a first image parameter related to generation of a first specimen image, and first effect information indicating a first effect applied to a first specimen captured in the first specimen image. Furthermore, the learning environment information may include first specimen attribute information that is specimen attribute information of the first specimen.

### 2.2. Example of Functional Configuration

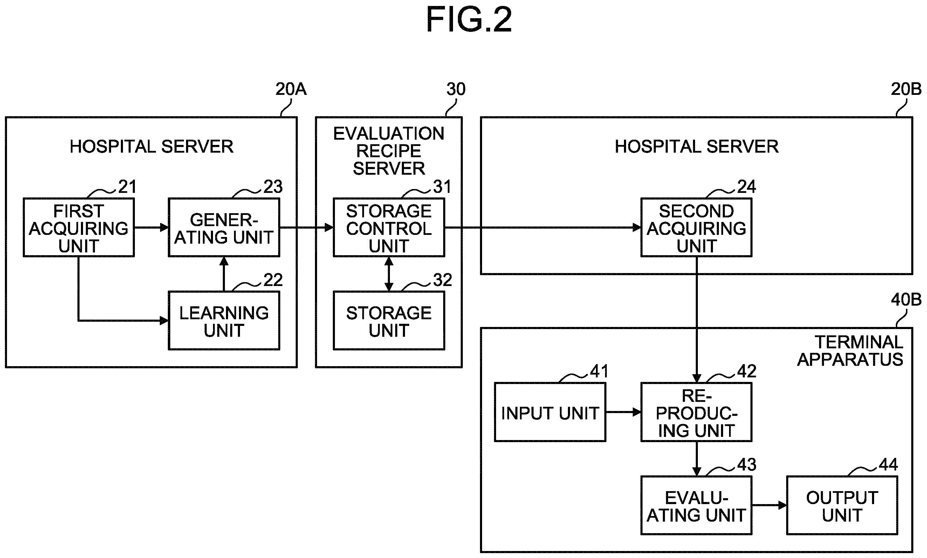

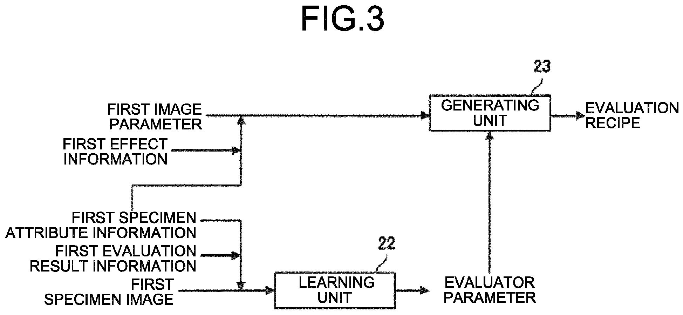

Hereinafter, an example of a functional configuration of the diagnostic system 1 according to the embodiment will be described, while reference is made to FIG. 2 and FIG. 3.

FIG. 2 is a diagram illustrating the example of the functional configuration of the diagnostic system 1 according to the embodiment. As illustrated in FIG. 2, the hospital server 20A includes a first acquiring unit 21, a learning unit 22, and a generating unit 23. The evaluation recipe server 30 includes a storage control unit 31 and a storage unit 32. The hospital server 20B includes a second acquiring unit 24. The terminal apparatus 40B includes an input unit 41, a reproducing unit 42, an evaluating unit 43, and an output unit 44.

FIG. 3 is a diagram illustrating flows of information related to generation of an evaluation recipe according to the embodiment. As illustrated in FIG. 3, first specimen attribute information, first evaluation result information, and first specimen images are input to the learning unit 22, and an evaluator parameter is generated. Furthermore, a first image parameter, first effect information, and the first specimen attribute information, as well as the evaluator parameter are input to the generating unit 23, and an evaluation recipe is generated.

### 2.2. 1. Example of Functional Configuration of Hospital Server 20A

### (1) First Acquiring Unit 21

The first acquiring unit 21 has a function of acquiring various types of information for generation of an evaluation recipe. Specifically, the first acquiring unit 21 acquires a first specimen image, and meta-information of the first specimen image. The acquired meta-information of the first specimen image is a first image parameter, first effect information, first specimen attribute information, and first evaluation result information. These pieces of information are acquired from, for example, the imaging device 10A, the terminal apparatus 40A, or an information system in the first hospital.

### (2) Learning Unit 22

The learning unit 22 has a function of learning an evaluator. Specifically, based on first specimen images of first specimens to which a first effect has been applied, the first specimen images having been captured by the imaging device 10A, the learning unit 22 learns an evaluator parameter of an evaluator that performs evaluation of a first specimen. For example, based on training data having first specimen images serving as data and first evaluation result information serving as labels, the learning unit 22 learns an evaluator parameter. In this case, an evaluator to which a specimen image is input and which outputs evaluation result information is learnt. Or, based on training data having data that are specimen images and at least a part of first specimen attribute information and labels that are first evaluation result information, the learning unit 22 may learn an evaluator parameter. In this case, an evaluator to which a specimen image and at least a part of specimen attribute information are input and which outputs evaluation result information is learnt. For example, an evaluator, to which a specimen image and the age and sex of a patient who is a sampling source of a specimen captured in the specimen image are input, and which outputs annotation information indicating a tumor region, is learnt.

The learning unit 22 learns an evaluator parameter for each learning environment. That is, the learning unit 22 learns an evaluator parameter, based on first specimen images having a learning environment in common (that is, the same learning environment). Specifically, based on training data having at least a part of their first image parameters, first effect information, and first specimen attribute information in common, the learning unit 22 learns an evaluator parameter. For example, based on plural first specimen images having, in common, identification information and a magnification setting of the imaging device 10A, the learning unit 22 learns an evaluator parameter. Furthermore, for example, based on first specimen images of plural patients having common gene expression tendencies in their genome information, the learning unit 22 learns an evaluator parameter. In any of these cases, by use of an evaluator parameter in an evaluation environment, the evaluator parameter having been learnt in a learning environment equivalent to the evaluation environment (for example, in the same environment), the evaluation accuracy is able to be improved.

Classifications of staining, such as HE and IHC, are able to be identified comparatively easily by image recognition. However, the types of antibodies are difficult to be identified by image recognition. Therefore, an evaluator parameter is learnt for each type of antibody, an evaluator parameter, which has been learnt in a learning environment where the type of antibody used is the same as that in the evaluation environment, is used in the evaluation, and the evaluation accuracy is thereby able to be improved.

Upon learning, first specimen images are generated with different image parameters for the same first specimen and used for the learning, desirably. For example, desirably, the same first specimen is captured by a plurality of the imaging devices 10A different from each other, or plural kinds of image processing (color correction and/or scaling) are applied to a captured image. As a result, evaluator parameters are able to be learnt in plural different learning environments, based on the same first specimen.

Different evaluator recipes may be learnt from the same training data. For example, plural evaluator recipes with different numbers of neural network layers may be learnt from the same training data.

### (3) Generating Unit 23

The generating unit 23 has a function of generating an evaluation recipe. The generating unit 23 generates an evaluation recipe by associating between an evaluator parameter learnt by the learning unit 22 and learning environment information indicating a learning environment for the evaluator parameter. The generating unit 23 transmits the evaluation recipe to the evaluation recipe server 30 to store the evaluation recipe in the evaluation recipe server 30.

An evaluation recipe may be accompanied by information other than learning environment information. Examples of the accompanying information include information related to the first specimen images that have been used as the training data. For example, an evaluation recipe may include, as accompanying information, pieces of meta-information of plural first specimen images that have been used in learning. An evaluation recipe desirably does not include personal information, such as the name of a patient who is the sampling source of the first specimen captured in first specimen images used in learning.

Table 1 has, listed therein, examples of evaluation recipes generated. As listed in Table 1, each evaluation recipe includes learning environment information, an evaluator parameter, and accompanying information. For example, Recipe A includes an evaluator parameter learnt based on first specimen images acquired by imaging of a first specimen, to which HE staining has been applied, and which has been sampled from a liver, at a magnification of "20 times" with the imaging device 10A that is "Device A".

**Table 1**

| Identif ication informa tion | Learning environment information | | | | Evaluator parameter | Accompa nying informa tion | |
|---|---|---|---|---|---|---|---|
| | Image parameter | | Specimen attribute information : type of organ | Effect information : staining information and antibody information | | | |
| | Type of imaging device | Magni ficat ion | | | | Age | ... |
| Recipe A | Device A | 20 × | Liver | HE | Parameter A | 60 years old | ... |
| Recipe B | Device A | 20 × | Pancreas | HE | Parameter B | 54 years old | ... |
| Recipe C | Device B | 10 × | Stomach | IHC-HER2 | Parameter C | 36 years old | ... |
| Recipe D | Device B | 20 × | Liver | HE | Parameter D | 72 years old | ... |
| Recipe E | Device B | 20 × | Mammary gland | IHC-HER2 | Parameter E | 40 years old | ... |

### 2.2. 2. Example of Functional Configuration of Evaluation Recipe Server 30

### (1) Storage Control Unit 31

The storage control unit 31 has a function of performing storage of information into the storage unit 32, and management of information stored in the storage unit 32. For example, the storage control unit 31 stores an evaluation recipe received from the hospital server 20A, into the storage unit 32. Furthermore, the storage control unit 31 retrieves, from the storage unit 32, an evaluation recipe corresponding to a request from the hospital server 20B, and transmits the evaluation recipe to the hospital server 20B. For example, the storage control unit 31 receives information indicating an evaluation environment, from the hospital server 20B, and transmits, to the hospital server 20B, an evaluation recipe including an evaluator parameter learnt in a learning environment equivalent to the evaluation environment.

### (2) Storage Unit 32

The storage unit 32 is a storage medium that stores therein various types of information. The storage unit 32 stores therein an evaluation recipe acquired from the hospital server 20A. For example, the storage unit 32 stores therein the evaluation recipes listed in Table 1.

### 2.2.3. Example of Functional Configurations of Hospital Server 20B and Terminal Apparatus 40B

### (1) Second Acquiring Unit 24

The second acquiring unit 24 acquires an evaluation recipe stored in the evaluation recipe server 30. Based on an evaluation environment of a second specimen image, the second acquiring unit 24 acquires an evaluation recipe. The acquisition of the evaluation recipe by the second acquiring unit 24 will be described later in detail.

### (2) Input Unit 41

The input unit 41 has a function of receiving input of various types of information. For example, the input unit 41 receives an operation for selection of an evaluation recipe by an employee of the second hospital.

### (3) Reproducing Unit 42

The reproducing unit 42 has a function of reproducing an environment equivalent to a learning environment, based on an evaluation recipe acquired by the second acquiring unit 24, the environment serving as an evaluation environment for evaluation of a second specimen. For example, the reproducing unit 42 performs: application of a second effect to a second specimen, the second effect being the same as a first effect indicated by first effect information; and/or generation of a second specimen image with a second image parameter equivalent to a first image parameter. The reproduction of the evaluation environment equivalent to the learning environment by the reproducing unit 42 will be described later in detail.

### (4) Evaluating Unit 43

The evaluating unit 43 performs evaluation of a second specimen image by using an evaluator, to which an evaluator parameter included in an evaluation recipe has been applied. For example, for pathological diagnosis of cancers, the evaluating unit 43 may determine whether or not cancer cells are present in a second specimen. Furthermore, the evaluating unit 43 may determine a region where the cancer cells have been generated in the second specimen image. Moreover, the evaluating unit 43 may determine malignancy of the cancer cells. In addition, the evaluating unit 43 may determine a drug for treatment of the cancer cells.

### (5) Output Unit 44

The output unit 44 has a function of outputting various types of information. For example, the input unit 41 outputs information indicating a result of evaluation by the evaluating unit 43.

### 3. Details of Reproduction Processing

### 3.1. First Reproduction Processing

First reproduction processing is processing where: an evaluation recipe is acquired in a state where a second captured image has not been captured yet; and an evaluation environment equivalent to a learning environment is reproduced. At the time of acquisition of the evaluation recipe, a second effect may have been applied already, or may have not been applied yet.

In the first reproduction processing, at least second specimen attribute information is determinate because a second specimen that is a target to be evaluated is already present. However, a second image parameter is indeterminate because the second captured image has not been captured yet, and if the second effect has not been applied yet, second effect information is also still indeterminate. These indeterminate parameters are controlled for reproduction of the evaluation environment equivalent to the learning environment.

### (1) Downloading of Evaluation Recipe

Firstly, the second acquiring unit 24 acquires an evaluation recipe that enables reproduction of an evaluation environment equivalent to a learning environment. Being equivalent herein does not necessarily mean being identical (perfectly matching). For example, even when the image parameters are different, if that difference is able to be artificially compensated by image processing, the learning environment and the evaluation environment are able to be regarded as being equivalent to each other.

### Referring to First Image Parameter

The second acquiring unit 24 acquires an evaluation recipe that enables reproduction of a second image parameter equivalent to a first image parameter, by referring to first image parameters of evaluation recipes stored in the evaluation recipe server 30. For example, the second acquiring unit 24 acquires an evaluation recipe in which the imaging device 10A is of the same type as the imaging device 10B. Furthermore, even if the imaging device 10A is of a type different from that of the imaging device 10B, the second acquiring unit 24 acquires an evaluation recipe that enables that difference between the types to be compensated by image processing. For example, the second acquiring unit 24 acquires an evaluation recipe of the imaging device 10A if equivalent color appearance and magnification are able to be reproduced by color correction and/or scaling, even if the imaging device 10A is of a type different from that of the imaging device 10B and differs in color appearance and/or magnification from the imaging device 10B.

### Referring to First Specimen Attribute Information

The second acquiring unit 24 acquires an evaluation recipe having first specimen attribute information that is the same as second specimen attribute information by referring to first specimen attribute information of evaluation recipes stored in the evaluation recipe server 30. For example, the second acquiring unit 24 acquires an evaluation recipe including an evaluator parameter learnt for a first specimen sampled from an organ that is the same as that of a second specimen. As a result, an evaluation environment with the same specimen attribute information as the learning environment is able to be reproduced. Even if all items of the first specimen attribute information do not match those of the second specimen attribute information, the second acquiring unit 24 may acquire an evaluation recipe having first specimen attribute information partially matching the second specimen attribute information. In this case, an evaluation environment that is the same as a learning environment for a first specimen having specimen attribute information similar to that of the second specimen is able to be reproduced.

### Referring to First Effect Information

### When Effect Has Been Applied at Time of Acquisition of Evaluation Recipe

The second acquiring unit 24 acquires an evaluation recipe having first effect information that is the same as second effect information, by referring to first effect information of evaluation recipes stored in the evaluation recipe server 30. For example, the second acquiring unit 24 acquires an evaluation recipe including an evaluator parameter learnt based on first specimen images to which a first effect that is the same as a second effect has been applied. As a result, an evaluation environment with the same effect as the learning environment is able to be reproduced.

### When Effect Has Not Been Applied at Time of Acquisition of Evaluation Recipe

The second acquiring unit 24 acquires an evaluation recipe including first effect information that is adoptable as second effect information, by referring to first effect information of evaluation recipes stored in the evaluation recipe server 30. For example, the second acquiring unit 24 acquires an evaluation recipe including an evaluator parameter learnt based on first specimen images to which an effect that is able to be applied in the second hospital (for example, when the second hospital owns equipment for staining) has been applied. As a result, an evaluation recipe that is difficult to be reproduced in the second hospital in the first place is able to be avoided.

### (2) Reproducing Evaluation Environment Equivalent to Learning Environment

The reproducing unit 42 reproduces an environment corresponding to a learning environment indicated by an evaluation recipe, the environment serving as an evaluation environment for evaluation of a second specimen.

### Reproducing First Effect Information

### When Effect Has Been Applied at Time of Acquisition of Evaluation Recipe

The reproducing unit 42 does not perform any processing in particular for an effect.

### When Effect Has Not Been Applied at Time of Acquisition of Evaluation Recipe

The reproducing unit 42 performs support for application of a second effect that is the same as a first effect, to a second specimen. For example, the reproducing unit 42 causes the output unit 44 to output first effect information and supports an action where an employee of the second hospital applies a second effect that is the same as a first effect to a second specimen. As a result, the second effect that is the same as the first effect is able to be applied to the second specimen. If the second hospital is equipped with facilities enabling automatic application of stimulation to cells, the reproducing unit 42 may apply a second effect to a second specimen by controlling the facilities. In this case, a fully automatic evaluation system is able to be constructed, the fully automatic evaluation system being where a process is automatically executed and an evaluation result is output, the processing being from an effect to evaluation, when an evaluation recipe is downloaded at the second hospital in a state where the second specimen has been prepared.

### Reproducing Image Parameter

The reproducing unit 42 reproduces a second image parameter equivalent to a first image parameter. More simply, the reproducing unit 42 generates a second specimen image that appears in the same way as a first specimen image. As a result, how the second specimen image appears is able to be made the same as how the first specimen image appears.

### When Imaging Devices Are of Same Type

When the imaging device 10A and the imaging device 10B are of the same type, the reproducing unit 42 makes a second image parameter the same as a first image parameter included in an evaluation recipe. Specifically, the reproducing unit 42 sets, for the imaging device 10B, a second imaging parameter that is the same as a first imaging parameter included in an evaluation recipe. For example, the reproducing unit 42 sets, for the imaging device 10B, an automatic focusing setting, a magnification, an exposure time period, and a gamma correction value, which are indicated by the first imaging parameter. The reproducing unit 42 applies second image processing that is the same as first image processing indicated by a first image processing parameter, to a second specimen image captured by the imaging device 10B.

Specific examples will be described while reference is made to Table 2 below. As listed in Table 2, if the imaging device 10A and the imaging device 10B are each "Device A", the reproducing unit 42 sets, for the imaging device 10B, "Setting A" that is the same as a first imaging parameter, and does not apply image processing in particular just like for first image processing. Furthermore, when the imaging device 10A and the imaging device 10B are each "Device B", the reproducing unit 42 sets, for the imaging device 10B, "Setting B" that is the same as a first imaging parameter, and applies "Color Correction B" that is the same as first image processing, to a second specimen image.

**Table 2**

| First imaging parameter | | First image processing | Second imaging parameter | | Second image processing |
|---|---|---|---|---|---|
| Type of imaging device | Other | | Type of imaging device | Other | |
| Device A | Setting A | None | Device A | Setting A | None |
| Device B | Setting B | Color correction B | Device B | Setting B | Color correction B |

### When Imaging Devices Are Not of Same Type

If the imaging device 10A and the imaging device 10B are not of the same type, the reproducing unit 42 applies image processing to a second specimen image, the image processing being for compensating for a difference between a first imaging parameter of the imaging device 10A and a second imaging parameter of the imaging device 10B. The image processing may include, for example, at least one of color correction or scaling. For example, if the difference between the types of the imaging device 10A and the imaging device 10B is able to be compensated by color correction, the reproducing unit 42 applies color correction for compensating for the difference between the types, to the second specimen image.

Specific examples will be described while reference is made to Table 3 below. As listed in Table 3, if the imaging device 10A is "Device A" and the imaging device 10B is "Device B", the reproducing unit 42 sets, for the imaging device 10B, "Setting A" that is the same as a first imaging parameter, and applies second image processing, "Color correction B", that is different from first image processing, "None", to a second specimen image. Furthermore, if the imaging device 10A is "Device C" and the imaging device 10B is "Device D", the reproducing unit 42 sets, for the imaging device 10B, "Setting A" that is the same as a first imaging parameter and applies second image processing, "Color correction D", that is different from first image processing, "Color correction C", to a second specimen image. If the imaging device 10A is "Device E" and the imaging device 10B is "Device F", the reproducing unit 42 sets, for the imaging device 10B, "Setting A" that is the same as a first imaging parameter, and applies second image processing, "None", that is different from first image processing, "Color correction E", to a second specimen image.

**Table 3**

| First imaging parameter | | First image processing | Second imaging parameter | | Second image processing |
|---|---|---|---|---|---|
| Type of imaging device | Other | | Type of imaging device | Other | |
| Device A | Setting A | None | Device B | Setting A | Color correction B |
| Device C | Setting A | Color correction C | Device D | Setting A | Color correction D |
| Device E | Setting A | Color correction E | Device F | Setting A | None |

### (3) Evaluation

The evaluating unit 43 evaluates a second specimen image of a second specimen, the second specimen image having been captured by the imaging device 10B, in an evaluation environment reproduced as an environment corresponding to a learning environment, by using an evaluator to which an evaluator parameter included in an evaluation recipe has been applied. That is, the evaluating unit 43 applies the evaluator parameter included in the evaluation recipe, to the evaluator, inputs the second specimen image acquired by the above described reproduction processing performed by the reproducing unit 42, into the evaluator, and thereby acquires an evaluation result.

### 3.2. Second Reproduction Processing

Second reproduction processing is processing where an evaluation environment equivalent to a learning environment is reproduced in a state where a second effect has been applied and a second specimen image has been captured.

In second reproduction processing, a second specimen that is a target to be evaluated is present, a second effect has been applied thereto, a second specimen image has been captured, and thus at least second specimen attribute information, second effect information, and a second imaging parameter are determinate. In contrast, since image processing on the second specimen image is possible, a second image processing parameter is thus indeterminate. This indeterminate parameter may be controlled for reproduction of an evaluation environment equivalent to the learning environment.

### (1) Downloading of Evaluation Recipe

Firstly, the second acquiring unit 24 acquires an evaluation recipe with a learning environment equivalent to an evaluation environment. For example, the second acquiring unit 24 acquires an evaluation recipe including an evaluator parameter that has been learnt in a learning environment equivalent to a learning environment of a second specimen image. Being equivalent herein does not necessarily mean being identical (perfectly matching). For example, even if the image parameters are different, if that difference is able to be artificially compensated by image processing, the learning environment and the evaluation environment are able to be regarded as being equivalent to each other.

### Referring to First Image Parameter

The second acquiring unit 24 acquires an evaluation recipe with a first image parameter equivalent to a second image parameter by referring to first image parameters of evaluation recipes stored in the evaluation recipe server 30. For example, the second acquiring unit 24 acquires an evaluation recipe in which the imaging device 10A is of the same type as the imaging device 10B. Furthermore, even if the imaging device 10A and the imaging device 10B are of different types, the second acquiring unit 24 acquires an evaluation recipe that enables that difference between the types to be compensated by image processing. For example, the second acquiring unit 24 acquires an evaluation recipe of the imaging device 10A if equivalent color appearance and magnification are able to be reproduced by color correction and/or scaling, even if the imaging device 10A is of a type different from that of the imaging device 10B and differs in color appearance and/or magnification from the imaging device 10B.

### Referring to First Specimen Attribute Information

The second acquiring unit 24 acquires an evaluation recipe having first specimen attribute information that is the same as second specimen attribute information, by referring to first specimen attribute information of evaluation recipes stored in the evaluation recipe server 30. For example, the second acquiring unit 24 acquires an evaluation recipe including an evaluator parameter learnt for first specimens sampled from the same organ as a second specimen. As a result, an evaluation environment having the same specimen attribute information as the learning environment is able to be reproduced. Even if all items of the first specimen attribute information do not match those of the second specimen attribute information, the second acquiring unit 24 may acquire an evaluation recipe having the first specimen attribute information partially matching the second specimen attribute information. In this case, an evaluation environment that is the same as the learning environment for the first specimens having the specimen attribute information similar to that of the second specimen is able to be reproduced.

### Referring to First Effect Information

The second acquiring unit 24 acquires an evaluation recipe having first effect information that is the same as second effect information, by referring to first effect information of evaluation recipes stored in the evaluation recipe server 30. For example, the second acquiring unit 24 acquires an evaluation recipe including an evaluator parameter learnt based on first specimen images to which a first effect that is the same as a second effect has been applied. As a result, an evaluation environment having the same effect as the learning environment is able to be reproduced.

### (2) Reproducing Evaluation Environment

### Equivalent to Learning Environment

The reproducing unit 42 reproduces an environment corresponding to a learning environment indicated by an evaluation recipe, the environment serving as an evaluation environment for evaluation of a second specimen. Second reproduction processing is performed in a state where a second effect has been applied, a second imaging parameter has been set, and a second captured image has been captured. Therefore, basically, by the time the evaluation recipe is acquired, the evaluation environment equivalent to the learning environment has already been reproduced.

However, imaging parameters may be different, like when the imaging device 10A and the imaging device 10B are of different types. In this case, the reproducing unit 42 applies image processing to the second specimen image, the image processing being for compensating for the difference between the first imaging parameter of the imaging device 10A and the second imaging parameter of the imaging device 10B. This image processing may include, for example, at least one of color correction or scaling. In addition, the image processing may include arbitrary processing, such as luminance correction, rotation, and/or binarization. As a result, the appearance of the second specimen image is able to be made similar to the appearance of the first specimen image, and the evaluation accuracy for the second specimen is able to be improved.

### (3) Evaluation

The evaluating unit 43 evaluates a second specimen image of a second specimen to which a second effect has been applied, the second specimen image having been captured by the imaging device 10B, by using an evaluator to which an evaluator parameter learnt in a learning environment equivalent to an evaluation environment has been applied. That is, the evaluating unit 43 applies the evaluator parameter included in an evaluation recipe, to the evaluator, inputs the second specimen image acquired by the above described reproduction processing performed by the reproducing unit 42, into the evaluator, and thereby acquires an evaluation result.

### 4. Example of UI

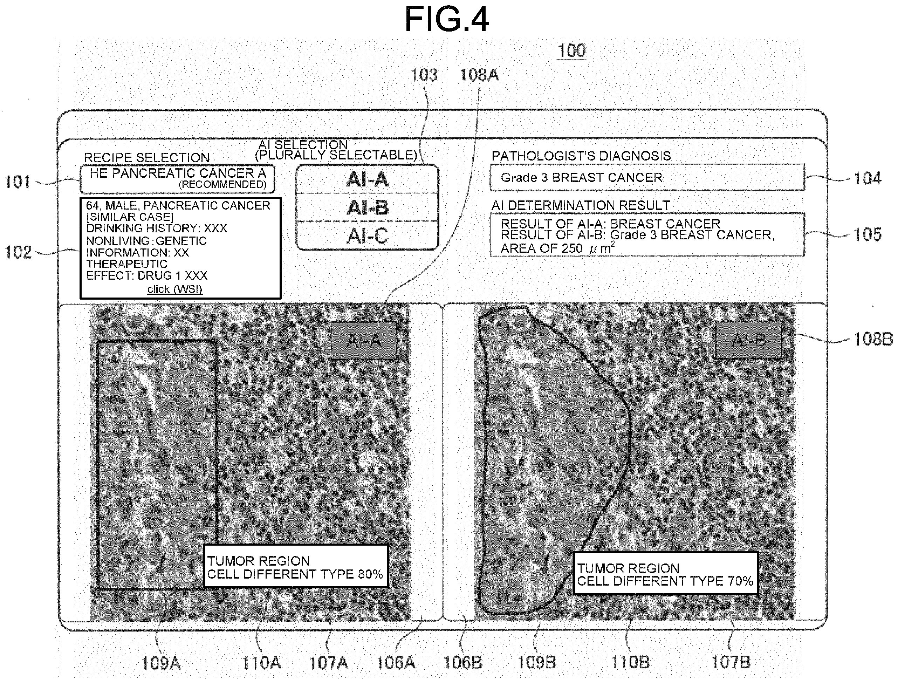

Hereinafter, an example of output information including a result of evaluation by the evaluating unit 43 will be described, the result being output by the output unit 44, while reference is made to FIG. 4.

FIG. 4 is a diagram illustrating an example of a user interface (UI) according to the embodiment. A UI 100 illustrated in FIG. 4 is output as an image by the output unit 44. A recipe selection field 101 has, displayed therein, identification information of an evaluation recipe that has been selected (that is, an evaluation recipe that has been downloaded). A similar case display field 102 has, displayed therein, information on a case similar to a case of a second specimen to be evaluated. An AI selection field 103 has, displayed therein, identification information of plural evaluator parameters included in evaluation recipes, and is for reception of a selection operation for an evaluator parameter to be applied to an evaluator. In the example illustrated in FIG. 4, "AI-A" and "AI-B" have been selected. A pathologist's diagnosis field 104 has, displayed therein, a diagnosis made by a pathologist for the second specimen to be evaluated. An AI determination result field 105 has, displayed therein, information indicating an evaluation result for the second specimen by an evaluator to which the evaluator parameter selected in the AI selection field 103 has been applied. An AI determination result detail display field 106 (106A and 106B) has the information displayed therein, the information indicating the evaluation result for the second specimen by the evaluator to which the evaluator parameter selected in the AI selection field 103 has been applied, the information being superimposed on a second specimen image 107 (107A and 107B). For example, identification information 108 (108A and 108B) of the evaluator parameter, annotation information 109 (109A and 109B) indicating a range of a tumor region, and information 110 (110A and 110B) indicating what the tumor is, are displayed, superimposed thereon.

By this UI 100, an employee of the second hospital is able to switch between evaluation recipes and between evaluator parameters, and compare evaluation results by different evaluator parameters by displaying them simultaneously. As a result, the employee of the second hospital is able to easily select appropriate evaluation recipe and evaluator parameter.

### 5. Flow of Processing

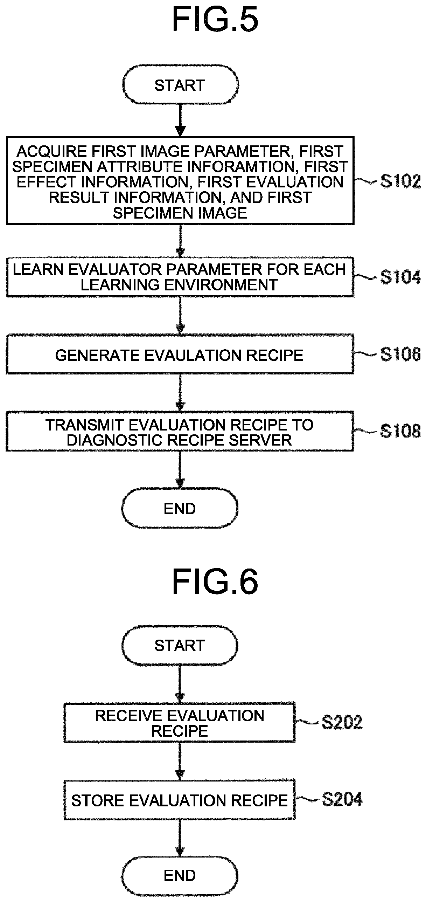

Hereinafter, an example of a flow of processing executed in the diagnostic system 1 according to the embodiment will be described by reference to FIG. 5 to FIG. 8.

### (1) Uploading Processing for Evaluation Recipe

FIG. 5 is a flow chart illustrating an example of a flow of uploading processing for an evaluation recipe, the uploading processing being executed in the hospital server 20A according to the embodiment. As illustrated in FIG. 5, firstly, the first acquiring unit 21 acquires first image parameters, first effect information, first specimen attribute information, first evaluation result information, and first specimen images (Step S102). Subsequently, based on training data including the first specimen images serving as data and the first evaluation result information serving as labels, the learning unit 22 learns an evaluator parameter for each learning environment (Step S104). Subsequently, the generating unit 23 generates an evaluation recipe by associating the evaluator parameter with information indicating the learning environment (for example, the first image parameter, first effect information, and/or first specimen attribute information that are common to the training data) (Step S106). The generating unit 23 then transmits the evaluation recipe generated, to the evaluation recipe server 30 (Step S108).

### (2) Storage Processing for Evaluation Recipe

FIG. 6 is a flow chart illustrating an example of a flow of storage processing for an evaluation recipe, the storage processing being executed in the evaluation recipe server 30 according to the embodiment. As illustrated in FIG. 6, firstly, the storage control unit 31 receives an evaluation recipe (Step S202). Subsequently, the storage unit 32 stores therein the evaluation recipe received (Step S204) .

### (3) First Reproduction Processing

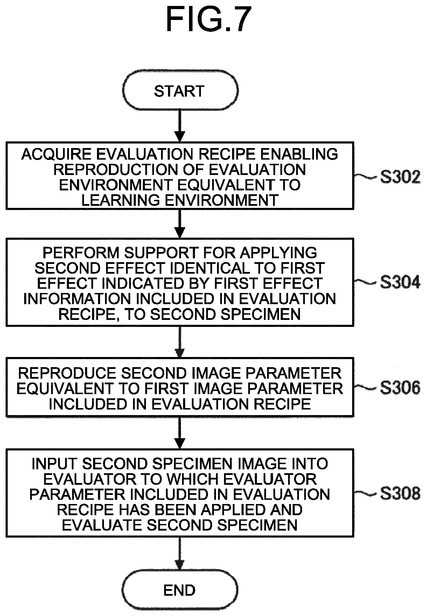

FIG. 7 is a flow chart illustrating an example of a flow of first reproduction processing executed in the hospital server 20B and the terminal apparatus 40B, according to the embodiment. As illustrated in FIG. 7, firstly, the second acquiring unit 24 acquires an evaluation recipe that enables reproduction of an evaluation environment equivalent to the learning environment (Step S302). For example, the second acquiring unit 24 refers to first image parameters, first specimen attribute information, and first effect information included in evaluation recipes stored in the evaluation recipe server 30, and acquires an evaluation recipe that enables reproduction of equivalent second image parameter, second specimen attribute information, and second effect information. Subsequently, the reproducing unit 42 performs support for applying a second effect to a second specimen, the second effect being the same as the first effect indicated by the first effect information included in the acquired evaluation recipe (Step S304). Next, the reproducing unit 42 reproduces the second image parameter equivalent to the first image parameter included in the evaluation recipe acquired (Step S306). As a result, a second specimen image generated in an evaluation environment equivalent to the learning environment is acquired. The evaluating unit 43 then evaluates the second specimen by inputting the second specimen image into an evaluator to which the evaluator parameter included in the evaluation recipe has been applied (Step S308).

### (4) Second Reproduction Processing

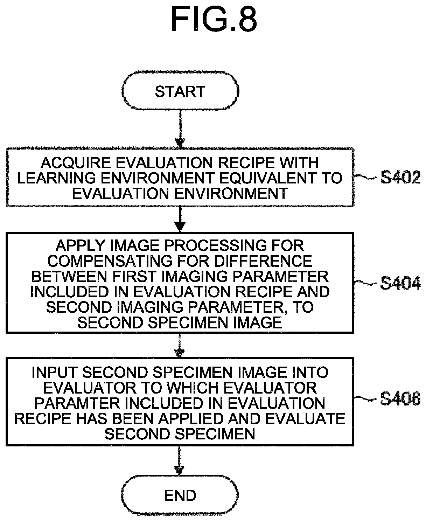

FIG. 8 is a flow chart illustrating an example of a flow of second reproduction processing executed in the hospital server 20B and the terminal apparatus 40B according to the embodiment. Before this flow is executed, a second effect has been applied to a second specimen, and a second specimen image has been captured. As illustrated in FIG. 8, firstly, the second acquiring unit 24 acquires an evaluation recipe with a learning environment equivalent to an evaluation environment (Step S402). For example, the second acquiring unit 24 refers to first image parameters, first specimen attribute information, and first effect information included in evaluation recipes stored in the evaluation recipe server 30, and acquires an evaluation recipe having a first image parameters, first specimen attribute information, and first effect information equivalent to a second image parameter, second specimen attribute information, and second effect information. Subsequently, the reproducing unit 42 applies image processing to the second specimen image, the image processing being for compensating for a difference between the first imaging parameter included in the evaluation recipe and a second imaging parameter (Step S404). Next, the evaluating unit 43 evaluates the second specimen by inputting the second specimen image into an evaluator to which the evaluator parameter included in the evaluation recipe has been applied (Step S406).

### 6. Application Examples

Examples in which the disclosed technique is applied to pathological diagnosis for cancers have been described above, but the disclosed technique is not necessarily applied to such examples. Application examples for the above described technique will be described below.

### (1) Drug Evaluation

The disclosed technique is applicable to drug evaluation.

In drug evaluation, states of cells after administration of drugs are observed. An effect in drug evaluation is administration of a drug. In drug evaluation, effects of a drug are proved by repetition of the same examination. For example, in drug evaluation where myocardial cells are used, effects of a drug are evaluated by beating analysis of myocardial cells. In this drug evaluation, the effects of the drug are evaluated based on time intervals and magnitude of the beating.

An evaluator in the drug evaluation outputs, for example, the effects of the drug, based on the time intervals and amplitude of the beating. An evaluation recipe in the drug evaluation includes drug information serving as information indicating the learning environment, and also the evaluator parameter of the evaluator.

### (2) Quality Evaluation Upon Cultivation of iPS cells

The disclosed technique is applicable to quality evaluation upon cultivation of iPS cells.

In quality evaluation upon cultivation of iPS cells, their capability of differentiation into an intended tissue or organ is evaluated. An effect in the quality evaluation upon cultivation of iPS cells is transfer of a gene and a protein or drug treatment. In the quality evaluation upon cultivation of iPS cells, whether or not tumor development due to genomic damage or undifferentiated cells is present is evaluated. Furthermore, in a cell cultivation process, iPS cells and non-iPS cells are determined by use of images. For intended cells to be found, an evaluator for image recognition may be used. For iPS cells, long-term monitoring is needed, and thus development of quality prediction technology using images may be considered.

Based on images at the time of cultivation, the evaluator for the quality evaluation upon cultivation of iPS cells evaluates: whether or not tumor development due to genomic damage or undifferentiated cells is present; whether the cells are iPS cells or non-iPS cells; and/or the prospective quality. The evaluation recipe for the quality evaluation upon cultivation of iPS cells includes information on transfer of a gene and a protein or on drug treatment, the information serving as information indicating the learning environment, and further includes the evaluator parameter of the evaluator.

### (3) Transplant Determination

The disclosed technique is applicable to transplant determination for cells in regenerative medicine. In this case, an evaluator evaluates possibility of transplant, based on an image of the cells.

### 7. Other Embodiments

### 7.1. Modified Examples of Evaluation Recipe

In the above described example, an evaluation recipe includes an evaluator parameter and learning environment information indicating a learning environment for the evaluator parameter. However, evaluation recipes are not limited to this example. For example, an evaluation recipe may include case information and evaluation result information. Case information indicates information related to symptoms of a disease and information related to a name of the disease. For example, case information indicates information related to a case or a disease name, such as prostate cancer, colon cancer, breast cancer, or lung cancer. Evaluation result information indicates the type and content of a diagnosis evaluated by the evaluator. An evaluation recipe including case information and evaluation result information may be referred to as a "diagnostic recipe" to distinguish it from the evaluation recipes described above. However, diagnostic recipes are a concept included in evaluation recipes.

An example of evaluation result information will be described below. For example, evaluation result information is information related to a result of determination in which whether a tumor is "cancerous" or" non-cancerous" is determined. Specifically, evaluation result information is information related to a result of determination in which whether or not a tumor is cancerous is determined with "1 (cancerous)" or "0 (non-cancerous)". In this case, information indicating a result of determination of whether or not cancer is present is the information output by an evaluator. Such an evaluator is able to be used in screening for determination of whether or not cancer is present. For example, evaluation result information is information related to an annotation result indicating a tumor region. In this case, the information indicating the annotation result indicating the tumor region is the information output by an evaluator. Such an evaluator is able to be used for confirmation or reconfirmation of a tumor region. For example, evaluation result information is information related to a result of classification of a tumor into a class. In this case, information indicating the result of the classification of the tumor into a class is the information output by an evaluator. Such an evaluator is able to be used for confirmation or reconfirmation of classification of the tumor into the class. For example, evaluation result information is information related to the shape, morphology, and area of a tumor, and a position of the cells or tissue. In this case, information indicating the shape, morphology, and area of the tumor, or information related to the position of the cells or tissue is the information output by an evaluator. Such an evaluator is able to be used as auxiliary information for diagnosis. Such an evaluator is able to be used as an auxiliary tool for cancer genomic diagnosis.

Examples of a diagnostic recipe include information like "diagnostic recipe 1 for prostate cancer" = "case information: prostate cancer" + "evaluation result information: cancer/non-cancer determination". Furthermore, other examples of a diagnostic recipe includes information like "diagnostic recipe 2 for colon cancer" = "case information: colon cancer" + "evaluation result information: annotation result indicating tumor region".

### 7.2. Types of Correction

With respect to the embodiment above, some examples of learning environment information included in evaluation recipes have been described. However, learning environment information included in an evaluation recipe is not limited to the above examples. For example, with respect to the embodiment above, an example where an imaging parameter is included in an evaluation recipe has been described, the imaging parameter being an example of device information related to an imaging device. This imaging parameter may include, in addition to a magnification mentioned above, color information related to a color of the image, or information related to the definition of the image. Furthermore, an evaluation recipe may include, not only color information of device information, but also staining information of the specimen and/or color information of organ information that is an example of specimen attribute information. This is because the color of an image may differ according to, not only the device, but also: the staining of the specimen; or the organ. Furthermore, the magnification, color, and definition of an image being different according to the imaging device, staining, or organ will hereinafter be referred to as having variation, as appropriate. A method of reducing the variation by correcting the magnification, color, and definition, of an image will be described below.

### 7.3. Outline of Correction Processing

With respect to the embodiment above, an example in which the second hospital performs evaluation processing in an evaluation environment according to a learning environment at the first hospital has been described. For example, the hospital server 20A at the first hospital generates an evaluation recipe and uploads the evaluation recipe to the evaluation recipe server 30. The hospital server 20B of the second hospital then acquires the evaluation recipe generated by the hospital server 20A from the evaluation recipe server 30. With respect to the embodiment above, an example in which the second hospital performs, based on an evaluation recipe, evaluation according to the learning environment at the first hospital has been described. For example, with respect to the embodiment above, an example in which the imaging device 10B generates a second specimen image based on an evaluation recipe, an example in which the reproducing unit 42 reproduces, based on an evaluation recipe, an environment equivalent to the learning environment, and an example in which the evaluating unit 43 performs evaluation by using an evaluator parameter included in an evaluation recipe have been described.

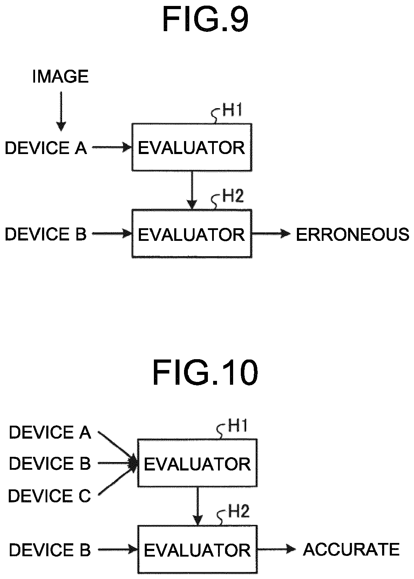

However, the method of making an evaluation environment and a learning environment the same is not limited to the above described embodiment. With respect to this point, patterns of methods of making an evaluation environment and a learning environment the same, the patterns including also the examples described with respect to the embodiment above, will be described by use of FIG. 9 to FIG. 12. For FIG. 9 to FIG. 12, a case where the definition is corrected will be described as an example. For FIG. 9 to FIG. 12, the case where the definition is corrected will be described as an example, but the same applies to a case where the magnification and/or color are/is corrected.

Hereinafter, in FIG. 9 to FIG. 12, an evaluator generated at the time of learning will be referred to as an "evaluator H1". Furthermore, hereinafter, in FIG. 9 to FIG. 12, an evaluator for evaluation by use of an evaluator will be referred to as an "evaluator H2".

### 7.4. Types of Correction Processing

FIG. 9 illustrates a case where a second specimen image captured by the imaging device 10B is evaluated by use of an evaluator generated based on first specimen images captured by the imaging device 10A. In this case, since the imaging device 10A and the imaging device 10B are different devices, the specimen images have different definitions, and the second specimen image may be unable to be evaluated appropriately. This is because the imaging devices differ in their device performance and imaging conditions, and thus the specimen images captured by the imaging device 10A and the imaging device 10B have definitions different between the imaging devices. For example, even if the same specimen is captured by the imaging device 10A and the imaging device 10B, a first specimen image and a second specimen image may have different definitions. That is, because an evaluator generated based on first specimen images captured by the imaging device 10A regards the first specimen images captured by the imaging device 10A as correct answer information, even if the evaluator is applied to a second specimen image captured by the imaging device 10B different from the imaging device 10A, variation may be caused in the specimen images between the imaging devices and appropriate evaluation may thus be unable to be performed. In other words, the evaluator generated based on the first specimen images captured by the imaging device 10A is for appropriately evaluating a first specimen image captured by the imaging device 10A, and does not necessarily appropriately evaluate a second specimen image captured by the imaging device 10B. Therefore, if whether or not a second specimen image captured by the imaging device 10B includes information related to a lesion is estimated by use of an evaluator generated based on first specimen images captured by the imaging device 10A, a result of the estimation may be erroneous. Appropriately evaluating means, for example, evaluating highly accurately. A case where a specimen image is corrected for correction of an error between imaging devices will be described below. FIG. 10 to FIG. 12 illustrate cases where definitions of specimen images are corrected.

FIG. 10 illustrates a case where an evaluator is generated based on specimen images captured by plural imaging devices. In FIG. 10, evaluators respectively corresponding the imaging devices are individually generated. Specifically, in FIG. 10, an evaluator for the imaging device 10A is generated based on first specimen images captured by the imaging device 10A, an evaluator for the imaging device 10B is generated based on second specimen images captured by the imaging device 10B, and an evaluator for an imaging device 10C is generated based on specimen images (hereinafter, referred to as "third specimen images", as appropriate) captured by the imaging device 10C. For example, highly accurate evaluation is enabled by use of the evaluators for these imaging devices, like: in a case where a first specimen image captured by the imaging device 10A is evaluated, the evaluator for the imaging device 10A is used; in a case where a second specimen image captured by the imaging device 10B is evaluated, the evaluator for the imaging device 10B is used; and in a case where a third specimen image captured by the imaging device 10C is evaluated, the evaluator for the imaging device 10C is used.

Furthermore, as illustrated in FIG. 10, instead of generating the evaluators corresponding respectively to the imaging devices, an evaluator corresponding to all of the imaging devices may be generated. Specifically, based on first specimen images captured by the imaging device 10A, second specimen image captured by the imaging device 10B, and third specimen images captured by the imaging device 10C, an evaluator corresponding to any of the first specimen images, second specimen images, and third specimen images may be generated. In this case, whether a first specimen image captured by the imaging device 10A is evaluated, a second specimen image captured by the imaging device 10B is evaluated, or a third specimen image captured by the imaging device 10C is evaluated, appropriate evaluation is enabled by use of the evaluator corresponding to all of these imaging devices.

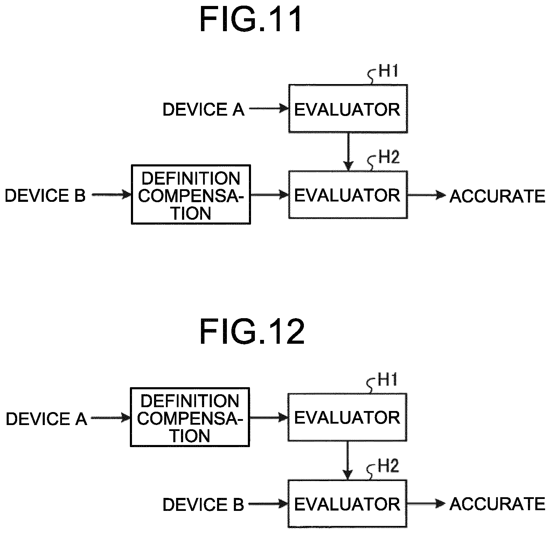

FIG. 11 illustrates a case where correction to a definition of a predetermined standard is performed upon evaluation. FIG. 11 illustrates a case where conformance to a learning environment is performed, like in the embodiment described above. In FIG. 11, a second specimen image is evaluated by use of an evaluator generated based on first specimen images captured by the imaging device 10A. Specifically, a corrected second specimen image is input to the evaluator generated based on the first specimen images captured by the imaging device 10A, the corrected second specimen image resulting from correction of a second specimen image captured by the imaging device 10B such that the second specimen image has a definition of a predetermined standard. More specifically, for evaluation of a second specimen image captured by the imaging device 10B by use of the evaluator generated based on the first specimen images captured by the imaging device 10A, the second specimen image captured by the imaging device 10B is corrected to a predetermined standard corresponding to the first specimen images. As a result, the second specimen image captured by the imaging device 10B is able to be evaluated without any error and the second specimen image is thus able to be evaluated more appropriately. However, the evaluation may take time because the second specimen image is input to the evaluator after being corrected to the standard definition. The case where conformance to the learning environment is performed has been described above.

Without being limited to the above described embodiment, learning may be performed by conformance to an evaluation environment. In other words, at the first hospital, a learning environment that is equivalent to an evaluation environment for evaluation of a second specimen may be reproduced and learning may be performed in the reproduced learning environment. FIG. 12 illustrates a case where learning is performed by conformance to an evaluation environment.

FIG. 12 illustrates a case where correction to a definition of a predetermined standard is performed upon generation of an evaluator. In FIG. 12, the evaluator is generated based on corrected first specimen images resulting from correction of first specimen images captured by the imaging device 10A to definitions of a predetermined standard. Specifically, for generation of the evaluator for evaluation of a second specimen image captured by the imaging device 10B, the first specimen images captured by the imaging device 10A are corrected to a predetermined standard corresponding to the second specimen image. As a result, the second specimen image captured by the imaging device 10B is able to be evaluated without any error and the second specimen image is thus able to be evaluated more appropriately. However, the generation of the evaluator may take time because the evaluator is generated after the correction to the standard definitions.

Hereinbefore, the cases where the definitions are corrected have been described as examples, but without being limited to definitions, similar processing may be performed when magnifications and/or colors of images are corrected.

### 7.5. Use of Evaluation Recipe

The cases where the generating unit 23 generates an evaluation recipe including an evaluator parameter and learning environment information have been described with respect to the embodiment above, but the embodiment is not limited to these examples. For example, the generating unit 23 may generate a diagnostic recipe that is an evaluation recipe including case information and evaluation result information.

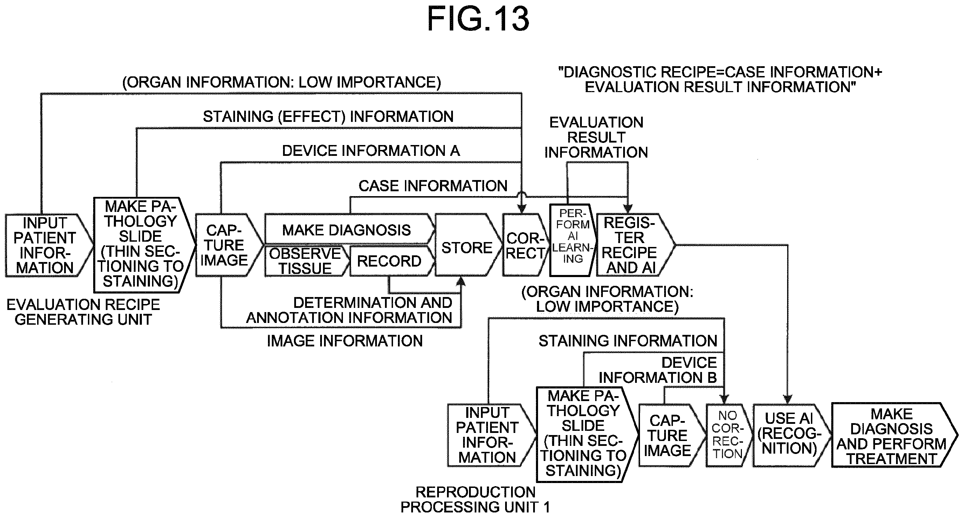

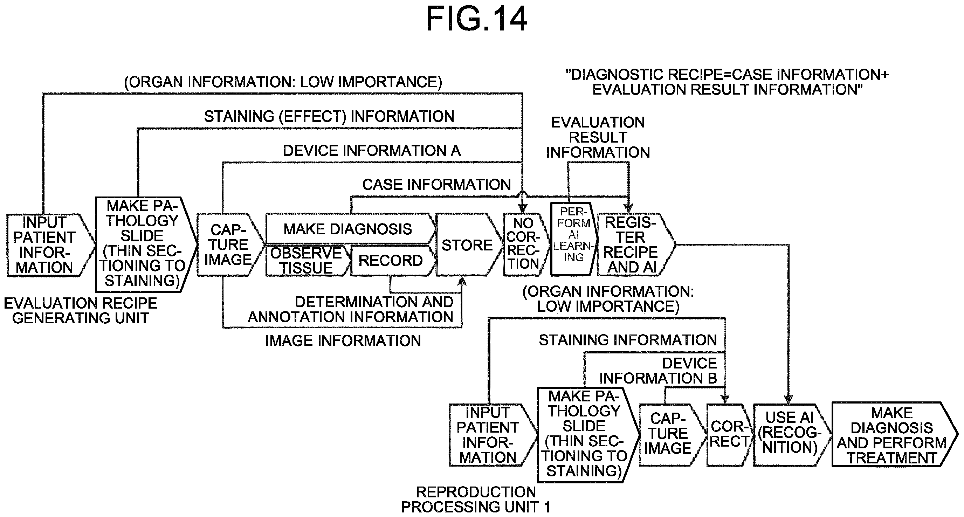

Processing where an evaluator is generated when correction is performed upon learning as illustrated in FIG. 12 will be described below. The generating unit 23 generates an evaluator with evaluation result information included in a diagnostic recipe, the evaluation result information serving as correct answer information for determination of lesions. For example, the generating unit 23 generates an evaluator with result information including determination results of determination of whether or not tumors are cancerous and annotation results indicating tumor regions, the result information being from evaluation result information included in a diagnostic recipe, the result information serving as correct answer information. In this case, the generating unit 23 generates the evaluator with information related to the content of evaluation, the information being from the evaluation result information included in the diagnostic recipe, the information being on, for example, cancer/non-cancer determination and annotations indicating the tumor regions, the information serving as an evaluator parameter. Furthermore, the generating unit 23 generates the evaluator with information that has been corrected based on information on a device, staining, an organ, and the like, the information serving as the evaluator parameter. For example, the generating unit 23 generates the evaluator with information including magnifications, colors, and definitions that have been corrected based on the information on the device, staining, and organ, to a predetermined standard, the information serving as the evaluator parameter. Hereinafter, a flow of information processing where an evaluation recipe is used will be described by use of FIG. 13 and FIG. 14.

There are two cases of information processing where an evaluation recipe is used. Specifically, there are: a case where correction is performed upon generation of an evaluator; and a case where correction is performed upon evaluation. Furthermore, the reproducing unit 42 may perform processing by using an evaluation recipe stored in the evaluation recipe server 30, or may perform processing by using information not stored in the evaluation recipe server 30. FIG. 13 and FIG. 14 illustrate a case where the reproducing unit 42 performs processing by using an evaluation recipe stored in the evaluation recipe server 30. Hereinafter, information processing in a case where correction is performed upon learning will be described by use of FIG. 13.

### 7.5.1. Information Processing Based on Correction upon Learning

FIG. 13 is a diagram illustrating an example of a procedure of information processing in a case where stored information is corrected by the generating unit 23 upon learning. Hereinafter, the example of the procedure of the information processing in the case where the stored information is corrected by the generating unit 23 upon learning will be described by use of FIG. 13. The generating unit 23 acquires patient information. For example, the generating unit 23 acquires patient information input by a pathologist. For example, the generating unit 23 acquires organ information. The generating unit 23 may acquire patient information that is not necessarily organ information and that may be any information related to the living body of the patient. For example, the generating unit 23 may acquire attribute information, such as the age, height, and sex of the patient.

The generating unit 23 acquires information related to a pathology slide. The generating unit 23 acquires information related to a pathology slide corresponding to patient information acquired. For example, the generating unit 23 acquires information related to a pathology slide resulting from thin sectioning and staining. For example, the generating unit 23 acquires staining (effect) information. A target to be stained may be anything related to a living body. For example, a target to be stained may be cells or blood.

The generating unit 23 acquires information related to a specimen image. For example, the generating unit 23 acquires information related to a specimen image of a pathology slide. For example, the generating unit 23 acquires information related to a specimen image of a target that has been stained. For example, the generating unit 23 acquires device information of an imaging device that has captured a specimen image.

The generating unit 23 acquires information related to a diagnosis. For example, the generating unit 23 acquires information related to a diagnosis based on a specimen image that has been captured. For example, the generating unit 23 acquires case information. For example, the generating unit 23 acquires annotation information. Diagnosis illustrated in FIG. 13 includes observation and recording. For example, the generating unit 23 acquires annotation information that has been recorded.

The generating unit 23 stores information related to a specimen image that has been acquired. For example, the generating unit 23 stores the specimen image into a predetermined storage unit. For example, the generating unit 23 stores annotation information.

The generating unit 23 performs correction, based on patient information, information related to a pathology slide, and information related to a pathological image. For example, the generating unit 23 performs correction, based on organ information, staining information, and device information. For example, the generating unit 23 is able to reduce variation due to the organ, by performing correction based on organ information. For example, the generating unit 23 is able to reduce variation due to the staining, by performing correction based on staining information. For example, the generating unit 23 is able to reduce variation due to the device, by performing correction based on device information. Furthermore, an error may be generated in the color due to the variation among the organs. According to the color of the organ and a predetermined reference color, the generating unit 23 performs correction. Furthermore, an error may be generated in the color due to variation in the staining. According to the color of the staining and a predetermined reference color, the generating unit 23 performs correction. Furthermore, an error may be generated in at least one of the color, magnification, and definition, according to the variation among the devices. The generating unit 23 performs correction according to at least one of a predetermined reference color, a predetermined reference magnification, and a predetermined reference definition. As a result, the generating unit 23 enables improvement in the accuracy of the evaluation. The generating unit 23 thus enables improvement in the accuracy of the evaluation by correcting at least one of the color, magnification, and definition.

Furthermore, the generating unit 23 may treat organ information as auxiliary information for correction. That is, the generating unit 23 corrects a specimen image by using organ information. For example, in detection of a cell nucleus, the generating unit 23 may use organ information as auxiliary information for correction if a tissue or the cell nucleus of the organ changes in color due to staining (for example, HE staining). Furthermore, in detection of a cell nucleus, the generating unit 23 may treat information on a mucous membrane, a hematopoietic system, and/or a salivary gland, as auxiliary information for correction.

The generating unit 23 performs learning by machine learning. For example, the generating unit 23 performs learning based on a neural network, such as deep learning. The generating unit 23 generates an evaluator based on patient information after correction, information related to a pathology slide after the correction, and information related to a specimen image after the correction. For example, the generating unit 23 may generate an evaluator with result information of evaluation result information, the result information serving as correct answer information. Learning according to the embodiment is not necessarily learning based on a neural network, such as deep learning, and may be any learning by machine learning. For example, learning according to the embodiment may be learning based on a random forest.

The generating unit 23 stores therein evaluators and evaluation recipes.

Based on processing similar to that by the generating unit 23, the reproducing unit 42 acquires organ information, staining information, and device information. The reproducing unit 42 requests the generating unit 23 for an evaluator corresponding to the information acquired. Based on the evaluator transmitted from the generating unit 23, the reproducing unit 42 specifies a pathological target. The reproducing unit 42 thus makes a diagnosis by using the evaluator generated by the generating unit 23.

### 7.5.2. Information Processing Based on Correction upon Evaluation

FIG. 14 is a diagram illustrating an example of a procedure of information processing in a case where information acquired by the reproducing unit 42 is corrected upon evaluation. Hereinafter, the example of the procedure of the information processing in the case where the information acquired by the reproducing unit 42 is corrected upon evaluation will be described by use of FIG. 14. Hereinafter, description of processing similar to that in FIG. 13 will be omitted as appropriate. Based on processing similar to that in FIG. 13, the generating unit 23 acquires patient information, information related to pathology slides, information related to specimen images, and information related to diagnoses. Furthermore, the generating unit 23 stores the information related to the specimen images and annotation information.

The generating unit 23 performs machine learning by using the information that has been acquired. The generating unit 23 performs learning, based on the patient information, the information related to the pathology slides, and the information related to the specimen images. The generating unit 23 stores an evaluator and an evaluation recipe.

Based on processing similar to that in FIG. 13, the reproducing unit 42 acquires organ information, staining information, and device information. The reproducing unit 42 corrects the organ information, staining information, and device information that have been acquired. For example, the reproducing unit 42 corrects the organ information, staining information, and device information, based on a predetermined standard.

The reproducing unit 42 requests the generating unit 23 for an evaluator corresponding to the information that has been corrected. Based on the evaluator transmitted from the generating unit 23, the reproducing unit 42 performs determination for a pathological target.

Information processing where an evaluation recipe is used has been described above. FIG. 13 and FIG. 14 illustrate the case where correction is performed at the time of either one of learning or evaluation, but correction may be performed upon both learning and evaluation. Furthermore, FIG. 13 and FIG. 14 illustrate the case where the generating unit 23 provides an evaluator to the reproducing unit 42, but the generating unit 23 may provide an estimation result output by use of an evaluator, to the reproducing unit 42. In this case, based on the estimation result provided, the reproducing unit 42 may perform determination for a pathological target.

### 7.6. Modified Examples of Specimen Attribute Information

According to the above described embodiment, specimen attribute information includes, for example: the age, sex, and age at the time of examination, of the patient; the type of organ of the sampling source of the specimen; the sampling method; the examination date; a result of pathological diagnosis; pathological findings; a thumbnail image of the specimen; and genome information of the specimen. However, specimen attribute information is not limited to this example. Specimen attribute information may include information related to ethnicity, such as the nationality and race of the patient. In this case, the generating unit 23 may generate an evaluation recipe with the information on the nationality and race of the patient, the information serving as patient information. Furthermore, specimen attribute information may include information related to the location of diagnosis, such as the hospital and country where the patient has been diagnosed. In this case, the generating unit 23 may generates an evaluation recipe with the information on the hospital and country where the patient has been diagnosed, the information serving as patient information.

The generating unit 23 may generate different evaluation recipes according to specimen attribute information. For example, the generating unit 23 may generate different evaluation recipes respectively for nationalities and races of patients. For example, the generating unit 23 may generate evaluation recipes based on patient information that differs among nationalities and races of patients respectively.

The generating unit 23 may generate different evaluators according to specimen attribute information. For example, the generating unit 23 may generate different evaluators respectively for nationalities and races of patients. For example, the generating unit 23 may generate evaluators based on patient information that differs among nationalities and races of patients. For example, the generating unit 23 may generate different evaluators respectively for nationalities and races of patients.

The generating unit 23 may transmit a corresponding evaluator to the reproducing unit 42, according to specimen attribute information corresponding to the ethnicity, such as the nationality and race of a patient to be evaluated, or corresponding to the location of diagnosis, such as the hospital and country where the patient has been diagnosed.

### 7.7. Generation of Combined Recipe

The case where an evaluator is generated by use of a predetermined evaluation recipe has been described with respect to the embodiment above, but the embodiment is not limited to this example. The generating unit 23 may generate a combined recipe that is a recipe resulting from combination of evaluation recipes. The generating unit 23 may generate an evaluator by using the combined recipe generated, and use the evaluator for diagnosis. For example, the generating unit 23 may generate a combined recipe if a specimen image includes plural pathological targets. Furthermore, the generating unit 23 may make a diagnosis, based on the information processing described above, with the generated combined recipe serving as a new evaluation recipe. For example, according to the combined recipe generated, the generating unit 23 may transmit an evaluator corresponding to the combined recipe, to the reproducing unit 42.

### 7.8. Modified Examples of Configuration

The diagnostic system 1 according to the embodiment is not limited to the example illustrated in FIG. 1, and may include a plurality of each of its components. Specifically, the diagnostic system 1 may include a plurality of the imaging devices 10 (a plurality of the imaging devices 10A and a plurality of the imaging devices 10B), a plurality of the hospital servers 20 (a plurality of the hospital servers 20A and a plurality of the hospital servers 20B), a plurality of the evaluation recipe servers 30, and a plurality of the terminal apparatuses 40 (a plurality of the terminal apparatuses 40A and a plurality of the terminal apparatuses 40B). Furthermore, the diagnostic system 1 according to the embodiment is not limited to the example illustrated in FIG. 2, and each of the components may include a plurality of its functions (for example, its processing units). For example, the hospital server 20 may include a plurality of the learning units 22 and a plurality of the generating units 23. Furthermore, an information processing system according to the embodiment may be implemented by a plurality of the diagnostic systems 1.

With respect to the embodiment above, the case where the hospital server 20 includes the first acquiring unit 21, the learning unit 22, the generating unit 23, and the second acquiring unit 24 has been described, but the embodiment is not limited to this example. The hospital server 20 may have a providing unit 25 that provides an evaluator. For example, the providing unit 25 provides an evaluator that has been generated by the generating unit 23. For example, the providing unit 25 provides an evaluator that has been acquired by the second acquiring unit 24. For example, the providing unit 25 transmits various types of information to the terminal apparatus 40. For example, the providing unit 25 provides an estimation result output by use of an evaluator.

### 7.9. Notation for User

With respect to the embodiment above, the case where diagnostic assistance is performed for a pathologist in making a diagnosis has been described, but a person who makes a diagnosis is not necessarily a pathologist, and may be anyone. For example, a person who makes a diagnosis may be a person related to a hospital, such as a doctor or a technical expert. Hereinafter, a person who makes a diagnosis will be referred to as a "user", as appropriate.

### 7.10. Notation for Medical Image

With respect to the embodiment above, the case where pathological diagnosis is assisted has been described, but the embodiment is not limited to pathological diagnosis, and medical diagnosis related to any medical care may be assisted. Furthermore, with respect to the embodiment above, the case where a specimen image is acquired has been described, but the embodiment is not limited to specimen images, and any medical image related to medical care may be acquired. In this case, according to the embodiment, an evaluator may be generated based on an evaluation recipe corresponding to medical images.

### 8. Example of Hardware Configuration

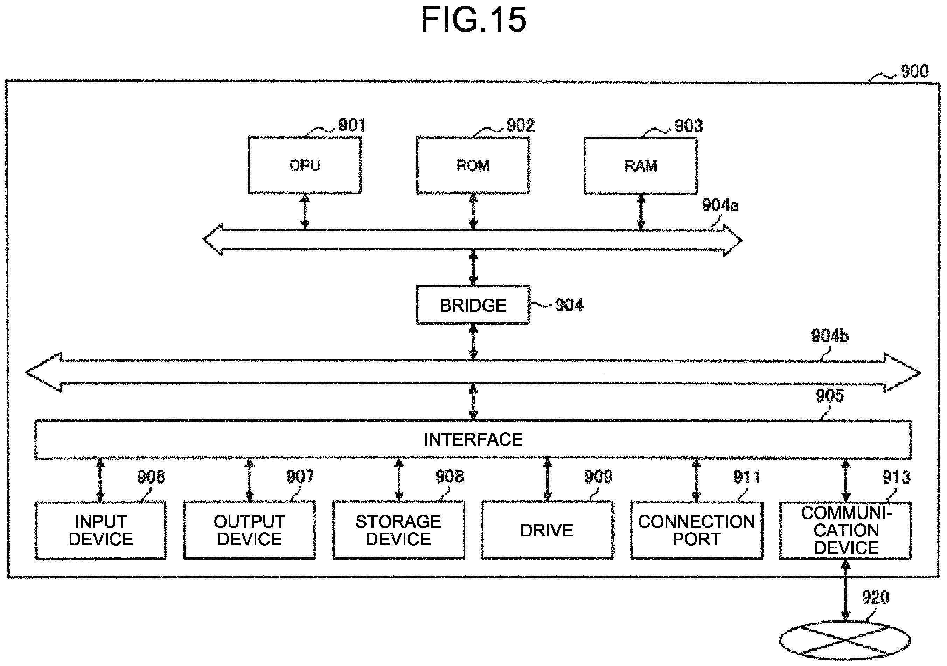

Lastly, a hardware configuration of an information processing apparatus according to the embodiment will be described by reference to FIG. 15. FIG. 15 is a block diagram illustrating an example of the hardware configuration of the information processing apparatus according to the embodiment. An information processing apparatus 900 illustrated in FIG. 15 may implement, for example, the hospital server 20A, the hospital server 20B, the evaluation recipe server 30, or the terminal apparatus 40B, which is illustrated in FIG. 2. Information processing by the hospital server 20A, the hospital server 20B, the evaluation recipe server 30, or the terminal apparatus 40B, according to the embodiment is implemented by cooperation between software and hardware described hereinafter.

As illustrated in FIG. 15, the information processing apparatus 900 includes a central processing unit (CPU) 901, a read only memory (ROM) 902, a random access memory (RAM) 903, and a host bus 904a. Furthermore, the information processing apparatus 900 includes a bridge 904, an external bus 904b, an interface 905, an input device 906, an output device 907, a storage device 908, a drive 909, a connection port 911, and a communication device 913. The information processing apparatus 900 may have, instead of the CPU 901, or in addition to the CPU 901, a processing circuit, such as an electric circuit, a DSP, or an ASIC.

The CPU 901 functions as an arithmetic processing device and a control device, and controls the overall operation in the information processing apparatus 900 according to various programs. Furthermore, the CPU 901 may be a microprocessor. The ROM 902 stores therein the programs, arithmetic parameters, and the like, which are used by the CPU 901. The RAM 903 temporarily stores therein a program used in execution by the CPU 901, parameters that change in the execution as appropriate, and the like. The CPU 901 may form, for example, the first acquiring unit 21, the learning unit 22, and the generating unit 23, which are illustrated in FIG. 2. Furthermore, the CPU 901 may form, for example, the storage control unit 31 illustrated in FIG. 2. In addition, the CPU 901 may form, for example, the second acquiring unit 24 illustrated in FIG. 2. Furthermore, the CPU 901 may form, for example, the reproducing unit 42 and the evaluating unit 43 illustrated in FIG. 2.

The CPU 901, the ROM 902, and the RAM 903 are connected to one another via the host bus 904a including a CPU bus or the like. The host bus 904a is connected to the external bus 904b, such as a peripheral component interconnect/interface (PCI) bus, via the bridge 904. The host bus 904a, the bridge 904, and the external bus 904b are not necessarily configured separately, and their functions may be implemented by a single bus.

The input device 906 is implemented by a device, into which information is input by a user, the device being, for example, any of: a mouse; a keyboard; a touch panel; a button or buttons; a microphone; a switch or switches; and a lever. Furthermore, the input device 906 may be, for example: a remote control device that uses infrared rays or other waves; or an externally connected device, such as a cellular phone or a PDA, which corresponds to operation of the information processing apparatus 900. Moreover, the input device 906 may include, for example, an input control circuit that generates an input signal, based on information input by the user by use of the above mentioned input means, and outputs the input signal to the CPU 901. The user of the information processing apparatus 900 is able to input various data to the information processing apparatus 900 and instruct the information processing apparatus 900 to perform processing and operation, by manipulating this input device 906. The input device 906 may form, for example, the input unit 41 illustrated in FIG. 2.

The output device 907 is formed of a device that is able to visually or aurally notify the user of acquired information. Examples of this device include: display devices, such as a CRT display device, a liquid crystal display device, a plasma display device, an EL display device, a laser projector, an LED projector, and a lamp; sound output devices, such as a speaker and a headphone; and printer devices. The output device 907 outputs, for example, results acquired by various types of processing performed by the information processing apparatus 900. Specifically, a display device visually displays the results acquired by the various types of processing performed by the information processing apparatus 900, in various formats, such as text, image, table, and graph formats. A sound output device converts an audio signal formed of reproduced sound data, acoustic data, or the like, into an analog signal, and aurally outputs the analog signal. The output device 907 may form, for example, the output unit 44 illustrated in FIG. 2.

The storage device 908 is a device for data storage, the device having been formed as an example of a storage unit of the information processing apparatus 900. The storage device 908 is implemented by, for example, a magnetic storage device, such as an HDD, a semiconductor storage device, an optical storage device, or a magneto-optical storage device. The storage device 908 may include a storage medium, a recording device that records data into the storage medium, a reading device that reads data from the storage medium, and a deleting device that deletes data recorded in the storage medium. This storage device 908 stores therein the programs executed by the CPU 901, various data, various types of data acquired from outside, and the like. The storage device 908 may form, for example, the storage unit 32 illustrated in FIG. 2.

The drive 909 is a storage media reader-writer, and is incorporated in or provided externally to the information processing apparatus 900. The drive 909 reads information recorded in a removable storage medium that has been inserted therein, such as a magnetic disk, an optical disk, a magneto-optical disk, or a semiconductor memory, and outputs the information to the RAM 903. Furthermore, the drive 909 is able to write information into the removable storage medium.

The connection port 911 is an interface connected to an external device, and serves as a connection port to the external device, the connection port enabling data transmission via, for example, a universal serial bus (USB).

The communication device 913 is a communication interface formed of, for example, a communication device for connection to a network 920. The communication device 913 is, for example, a communication card for a wired or wireless local area network (LAN), Long Term Evolution (LTE), Bluetooth (registered trademark), or a wireless USB (WUSB). Furthermore, the communication device 913 may be a router for optical communication, a router for an asymmetric digital subscriber line (ADSL), a modem for any of various types of communication, or the like. This communication device 913 is able to transmit and receive signals and the like according to a predetermined protocol, for example, TCP/IP, to and from, for example, the Internet or another communication device. The communication device 913 enables, for example, transmission and reception of signals between the devices illustrated in FIG. 2.

The network 920 is a wired or wireless transmission path for information transmitted from a device connected to the network 920. For example, the network 920 may include a public network, such as the Internet, a telephone network, or a satellite communication network; or any of various local area networks (LANs) and wide area networks (WANs) including Ethernet (registered trademark). Furthermore, the network 920 may include a leased line network, such as an internet protocol-virtual private network (IP-VPN).

An example of the hardware configuration that is able to implement functions of the information processing apparatus 900 according to the embodiment has been described above. Each of the above described components may be implemented by use of a versatile member, or may be realized by hardware specific to a function of that component. Therefore, a hardware configuration to be used may be modified, as appropriate, according to the technical level at the time the embodiment is implemented.

A computer program for implementing the functions of the information processing apparatus 900 according to the embodiment as described above may be made and installed on a PC or the like. Furthermore, a computer-readable recording medium having such a computer program stored therein may also be provided. The recording medium is, for example, a magnetic disk, an optical disk, a magneto-optical disk, or a flash memory. Moreover, without use of the recording medium, the above described computer program may be distributed via, for example, a network.

### 9. Conclusion

Hereinbefore, one embodiment of the present disclosure has been described in detail by reference to FIG. 1 to FIG. 15. As described above, based on first specimen images of a first specimen to which a first effect has been applied, the first specimen images having been captured by the imaging device 10A, the hospital server 20A according to the embodiment learns an evaluator parameter of an evaluator that performs evaluation of a first specimen. The hospital server 20A then generates an evaluation recipe including the evaluator parameter and learning environment information indicating a learning environment for the evaluator parameter and stores the evaluation recipe into the evaluation recipe server 30. The hospital server 20B according to the embodiment acquires an evaluation recipe stored in the evaluation recipe server 30. Based on the evaluation recipe acquired by the hospital server 20B, the terminal apparatus 40B reproduces an environment equivalent to a learning environment indicated by learning environment information of the evaluation recipe, the environment serving as an evaluation environment for evaluation of a second specimen of which an image is captured by the imaging device 10B different from the imaging device 10A. As a result, the second specimen is able to be evaluated by application of the evaluator parameter to an evaluator, the evaluator parameter having been learnt in the learning environment equivalent to the evaluation environment for the second specimen, and the evaluation accuracy for the second specimen is thus able to be improved.

In first reproduction processing, an evaluation recipe is acquired in a state where a second captured image has not been captured yet, and an evaluation environment equivalent to the learning environment is reproduced. The terminal apparatus 40B then evaluates a second specimen image of a second specimen, the second specimen image having been captured by the imaging device 10B in the evaluation environment that has been reproduced, by using an evaluator to which the evaluator parameter included in the evaluation recipe has been applied. In contrast, in second reproduction processing, an evaluation environment equivalent to the learning environment is reproduced in a state where a second effect has been applied and a second specimen image has been captured. The terminal apparatus 40B then evaluates the second specimen image of the second specimen to which the second effect has been applied, the second specimen image having been captured by the imaging device 10B, by using an evaluator to which the evaluator parameter learnt in the learning environment equivalent to the evaluation environment has been applied. In either case, since the learning environment and the evaluation environment become equivalent to each other, the accuracy of evaluation by the evaluator is able to be improved.

Protocols have been prepared for: setting of an imaging parameter for the imaging device 10B in an evaluation environment; application of a second effect; and the like, but the setting, the application, and the like are often implemented manually by humans. However, according to this embodiment, at least a part of reproduction processing based on an evaluation recipe is able to be implemented mechanically. Therefore, the workload on humans is able to be reduced, human error is able to be reduced, and the accuracy of evaluation is able to be improved.

In addition, by application of an evaluation recipe, replication study for theses is able to be facilitated, experiments are able to be reproduced accurately, and cells are able to be cultivated highly precisely.

A preferred embodiment of the present disclosure has been described in detail above by reference to the appended drawings, but the technical scope of the present disclosure is not limited to this example. It is evident that a person having ordinary skill in the technical field of the present disclosure can derive various modified examples or revised examples within the scope of the technical ideas written in the patent claims, and it is understood that these modified examples or revised examples also rightfully belong to the technical scope of the present disclosure.

For example, with respect to the embodiment above, the example where the components are mapped to the hospital server 20A, the evaluation recipe server 30, the hospital server 20B, and the terminal apparatus 40B as illustrated in FIG. 2 has been described, but the disclosed technique is not limited to this example. For example, the terminal apparatus 40B may include the second acquiring unit 24, and any other mapping may be allowed.

Furthermore, the processing described by use of the flow charts and sequence diagrams in this specification are not necessarily executed in the order illustrated therein. Any of the processing steps may be executed parallelly. Furthermore, an additional processing step may be adopted, or a part of the processing steps may be omitted.

Furthermore, the effects described in this specification are just explanatory or exemplary, and are not limiting. That is, the techniques according to the present disclosure may achieve other effects evident to those skilled in the art from the description in this specification, in addition to the above described effects or instead of the above described effects.

The following configurations also belong to the technical scope of the present disclosure.
(1) An information processing method, including:
   learning, based on a first specimen image of a first specimen to which a first effect has been applied, an evaluator parameter of an evaluator that performs evaluation of the first specimen, the first specimen image having been captured by a first imaging device;
   storing, into a storage medium, evaluation setting information including the evaluator parameter and learning environment information indicating a learning environment for the evaluator parameter;
   acquiring the evaluation setting information that has been stored in the storage medium; and
   reproducing, based on the evaluation setting information acquired, an environment equivalent to the learning environment, the environment serving as an evaluation environment for evaluation of a second specimen of which an image is captured by a second imaging device different from the first imaging device.
(2) The information processing method according to (1), wherein the learning includes learning the evaluator parameter for the learning environment.
(3) The information processing method according to (1), wherein the learning environment information includes a first image parameter related to generation of the first specimen image, effect information indicating the first effect, and first attribute information indicating an attribute of the first specimen.
(4) The information processing method according to (3), wherein the first image parameter includes a first imaging parameter of the first imaging device and first image processing information indicating image processing that has been applied to the first specimen image.
(5) The information processing method according to (3) or (4), wherein the effect information includes information indicating a type of staining and information indicating a type of an antibody used in the staining.
(6) The information processing method according to any one of (3) to (5), wherein the first attribute information includes a type of an organ of a sampling source of the first specimen.
(7) The information processing method according to any one of (3) to (6), wherein the first attribute information includes genome information of the first specimen.
(8) The information processing method according to any one of (3) to (7), further including:
   evaluating, at the evaluator to which the evaluator parameter included in the evaluation setting information has been applied, a second specimen image of the second specimen, the second specimen image having been captured by the second imaging device in the evaluation environment that has been reproduced.
(9) The information processing method according to (8), wherein the reproducing includes making a second image parameter related to generation of the second specimen image the same as the first image parameter included in the learning environment information.
(10) The information processing method according to (8), wherein the reproducing includes applying image processing to the second specimen image, the image processing being for compensating for a difference between a first imaging parameter of the first imaging device and a second imaging parameter of the second imaging device.
(11) The information processing method according to any one of (8) to (10), wherein the reproducing includes performing support for applying a second effect that is the same as the first effect, to the second specimen.
(12) The information processing method according to any one of (8) to (11), wherein the acquiring includes acquiring the evaluation setting information that enables reproduction of the evaluation environment equivalent to the learning environment.
(13) The information processing method according to any one of (3) to (7), further including:
   evaluating, at the evaluator to which the evaluator parameter that has been learnt in the learning environment equivalent to the evaluation environment has been applied, a second specimen image of the second specimen to which a second effect has been applied, the second specimen image having been captured by the second imaging device.
(14) The information processing method according to (13), wherein the acquiring includes acquiring the evaluation setting information according to which: the first imaging device and the second imaging device are of the same type; the first attribute information of the first specimen and second attribute information of the second specimen are the same; and the first effect and the second effect are the same.
(15) The information processing method according to (13) or (14), wherein the evaluating includes applying image processing to the second specimen image, the image processing being for compensating for a difference between a first imaging parameter of the first imaging device and a second imaging parameter of the second imaging device.
(16) The information processing method according to (15), wherein the image processing includes at least one of color correction or scaling.
(17) The information processing method according to any one of (1) to (16), including outputting identification information of the evaluation setting information, identification information of the evaluator parameter, and information indicating an evaluation result for the second specimen.
(18) The information processing method according to any one of (1) to (17), wherein the evaluating includes: determining whether or not cancer cells are present in the second specimen, identifying a region where the cancer cells have been generated in a second specimen image of the second specimen, determining malignancy of the cancer cells, and determining a drug for treatment of the cancer cells.
(19) An information processing method, including:
   learning, based on a first specimen image of a first specimen to which a first effect has been applied, an evaluator parameter of an evaluator that performs evaluation of the first specimen, the first specimen image having been captured by a first imaging device; and
   storing, into a storage medium, evaluation setting information including the evaluator parameter and learning environment information indicating a learning environment for the evaluator parameter.
(20) An information processing method, including:
   acquiring, from a storage medium, evaluation setting information including:
   an evaluator parameter of an evaluator that performs evaluation of a first specimen to which a first effect has been applied, the evaluator parameter having been learnt based on a first specimen image of the first specimen, the first specimen image having been captured by a first imaging device; and
      learning environment information indicating a learning environment for the evaluator parameter; and
   reproducing, based on the evaluation setting information acquired, an environment equivalent to the learning environment, the environment serving as an evaluation environment for evaluation of a second specimen of which an image is captured by a second imaging device different from the first imaging device.
(21) An information processing system, comprising:
   a first information processing apparatus including:
      a learning unit that learns, based on a first specimen image of a first specimen to which a first effect has been applied, an evaluator parameter of an evaluator that performs evaluation of the first specimen, the first specimen image having been captured by a first imaging device; and
      a generating unit that generates evaluation setting information including the evaluator parameter and learning environment information indicating a learning environment for the evaluator parameter;
   a second information processing apparatus including a storage medium storing therein the evaluation setting information; and
   a third information processing apparatus including an acquiring unit that acquires the evaluation setting information stored in the storage medium and reproduces, based on the evaluation setting information acquired, an environment equivalent to the learning environment, the environment serving as an evaluation environment for evaluation of a second specimen of which an image is captured by a second imaging device different from the first imaging device.
(22) An information processing apparatus, comprising:
   a learning unit that learns, based on a first specimen image of a first specimen to which a first effect has been applied, an evaluator parameter of an evaluator that performs evaluation of the first specimen, the first specimen image having been captured by a first imaging device; and
   a generating unit that generates evaluation setting information including the evaluator parameter and learning environment information indicating a learning environment for the evaluator parameter.
(23) An information processing apparatus, comprising:
   an acquiring unit that acquires, from a storage medium, evaluation setting information including:
      an evaluator parameter of an evaluator that performs evaluation of a first specimen to which a first effect has been applied; and
      learning environment information indicating a learning environment for the evaluator parameter, the evaluator parameter having been learnt based on a first specimen image of the first specimen, the first specimen image having been captured by a first imaging device; and
   a reproducing unit that reproduces, based on the evaluation setting information acquired, an environment equivalent to the learning environment, the environment serving as an evaluation environment for evaluation of a second specimen of which an image is captured by a second imaging device different from the first imaging device.
(24) An information processing system, comprising:
   an acquiring unit that acquires information transmitted from a terminal apparatus used by a pathologist according to operation by the pathologist, the information being related to a specimen image that has been captured for pathological diagnosis;
   a generating unit that generates an evaluator, based on an evaluation recipe that is evaluation setting information indicating a setting related to evaluation of the specimen image that has been corrected according to the information related to the specimen image acquired by the acquiring unit; and
   a providing unit that provides, to the terminal apparatus, information related to the evaluator generated by the generating unit, the evaluator being for evaluation of the specimen image.
(25) An information processing system, comprising:
   an acquiring unit that acquires information transmitted from a terminal apparatus used by a pathologist according to operation by the pathologist, the information being related to a specimen image that has been captured for pathological diagnosis;
   a generating unit that generates an evaluator, based on an evaluation recipe that is evaluation setting information indicating a setting related to evaluation of the specimen image, according to the information related to the specimen image, the information having been acquired by the acquiring unit; and
   a providing unit that provides, to the terminal apparatus, information related to the evaluator generated by the generating unit, the evaluator being for evaluation of the specimen image that has been corrected according to the evaluator.
(26) An information processing system, comprising:
   an acquiring unit that acquires information transmitted from a terminal apparatus used by a user according to operation by the user, the information being related to a medical image captured for diagnosis;
   a generating unit that generates an evaluator, based on an evaluation recipe that is evaluation setting information indicating a setting related to evaluation of the medical image that has been corrected according to the information related to the medical image, the information having been acquired by the acquiring unit; and
   a providing unit that provides, to the terminal apparatus, information related to the evaluator generated by the generating unit, the evaluator being for evaluation of the medical image.
(27) An information processing system, comprising:
   an acquiring unit that acquires information transmitted from a terminal apparatus used by a user according to operation by the user, the information being related to a medical image captured for diagnosis;
   a generating unit that generates an evaluator, based on an evaluation recipe that is evaluation setting information indicating a setting related to evaluation of the medical image, according to the information related to the medical image, the information having been acquired by the acquiring unit; and
   a providing unit that provides, to the terminal apparatus, information related to the evaluator generated by the generating unit, the evaluator being for evaluation of the medical image that has been corrected according to the evaluator.

### Reference Signs List

- 1: DIAGNOSTIC SYSTEM
- 10: IMAGING DEVICE
- 20: HOSPITAL SERVER
- 21: FIRST ACQUIRING UNIT
- 22: LEARNING UNIT
- 23: GENERATING UNIT
- 24: SECOND ACQUIRING UNIT
- 30: EVALUATION RECIPE SERVER
- 31: STORAGE CONTROL UNIT
- 32: STORAGE UNIT
- 40: TERMINAL APPARATUS
- 41: INPUT UNIT
- 42: REPRODUCING UNIT
- 43: EVALUATING UNIT
- 44: OUTPUT UNIT

## Claims

1. An information processing method, including:
learning, based on a first specimen image of a first specimen to which a first effect has been applied, an evaluator parameter of an evaluator that performs evaluation of the first specimen, the first specimen image having been captured by a first imaging device;
storing, into a storage medium, evaluation setting information including the evaluator parameter and learning environment information indicating a learning environment for the evaluator parameter;
acquiring the evaluation setting information that has been stored in the storage medium; and
reproducing, based on the evaluation setting information acquired, an environment equivalent to the learning environment, the environment serving as an evaluation environment for evaluation of a second specimen of which an image is captured by a second imaging device different from the first imaging device.

2. The information processing method according to claim 1, wherein the learning includes learning the evaluator parameter for the learning environment.

3. The information processing method according to claim 1, wherein the learning environment information includes a first image parameter related to generation of the first specimen image, effect information indicating the first effect, and first attribute information indicating an attribute of the first specimen.

4. The information processing method according to claim 3, wherein the first image parameter includes a first imaging parameter of the first imaging device and first image processing information indicating image processing that has been applied to the first specimen image.

5. The information processing method according to claim 3, wherein the effect information includes information indicating a type of staining and information indicating a type of an antibody used in the staining.

6. The information processing method according to claim 3, wherein the first attribute information includes a type of an organ of a sampling source of the first specimen.

7. The information processing method according to claim 3, wherein the first attribute information includes genome information of the first specimen.

8. The information processing method according to claim 3, further including:
evaluating, at the evaluator to which the evaluator parameter included in the evaluation setting information has been applied, a second specimen image of the second specimen, the second specimen image having been captured by the second imaging device in the evaluation environment that has been reproduced.

9. The information processing method according to claim 8, wherein the reproducing includes making a second image parameter related to generation of the second specimen image the same as the first image parameter included in the learning environment information.

10. The information processing method according to claim 8, wherein the reproducing includes applying image processing to the second specimen image, the image processing being for compensating for a difference between a first imaging parameter of the first imaging device and a second imaging parameter of the second imaging device.

11. The information processing method according to claim 8, wherein the reproducing includes performing support for applying a second effect that is the same as the first effect, to the second specimen.

12. The information processing method according to claim 8, wherein the acquiring includes acquiring the evaluation setting information that enables reproduction of the evaluation environment equivalent to the learning environment.

13. The information processing method according to claim 3, further including:
evaluating, at the evaluator to which the evaluator parameter that has been learnt in the learning environment equivalent to the evaluation environment has been applied, a second specimen image of the second specimen to which a second effect has been applied, the second specimen image having been captured by the second imaging device.

14. The information processing method according to claim 13, wherein the acquiring includes acquiring the evaluation setting information according to which: the first imaging device and the second imaging device are of the same type; the first attribute information of the first specimen and second attribute information of the second specimen are the same; and the first effect and the second effect are the same.

15. The information processing method according to claim 13, wherein the evaluating includes applying image processing to the second specimen image, the image processing being for compensating for a difference between a first imaging parameter of the first imaging device and a second imaging parameter of the second imaging device.

16. The information processing method according to claim 15, wherein the image processing includes at least one of color correction or scaling.

17. The information processing method according to claim 1, including outputting identification information of the evaluation setting information, identification information of the evaluator parameter, and information indicating an evaluation result for the second specimen.

18. The information processing method according to claim 1, wherein the evaluating includes: determining whether or not cancer cells are present in the second specimen, identifying a region where the cancer cells have been generated in a second specimen image of the second specimen, determining malignancy of the cancer cells, and determining a drug for treatment of the cancer cells.

19. An information processing method, including:
learning, based on a first specimen image of a first specimen to which a first effect has been applied, an evaluator parameter of an evaluator that performs evaluation of the first specimen, the first specimen image having been captured by a first imaging device; and
storing, into a storage medium, evaluation setting information including the evaluator parameter and learning environment information indicating a learning environment for the evaluator parameter.

20. An information processing method, including:
acquiring, from a storage medium, evaluation setting information including:
an evaluator parameter of an evaluator that performs evaluation of a first specimen to which a first effect has been applied, the evaluator parameter having been learnt based on a first specimen image of the first specimen, the first specimen image having been captured by a first imaging device; and
learning environment information indicating a learning environment for the evaluator parameter; and
reproducing, based on the evaluation setting information acquired, an environment equivalent to the learning environment, the environment serving as an evaluation environment for evaluation of a second specimen of which an image is captured by a second imaging device different from the first imaging device.

21. An information processing system, comprising:
an acquiring unit that acquires information transmitted from a terminal apparatus used by a pathologist according to operation by the pathologist, the information being related to a specimen image that has been captured for pathological diagnosis;
a generating unit that generates an evaluator, based on an evaluation recipe that is evaluation setting information indicating a setting related to evaluation of the specimen image that has been corrected according to the information related to the specimen image acquired by the acquiring unit; and
a providing unit that provides, to the terminal apparatus, information related to the evaluator generated by the generating unit, the evaluator being for evaluation of the specimen image.

22. An information processing system, comprising:
an acquiring unit that acquires information transmitted from a terminal apparatus used by a pathologist according to operation by the pathologist, the information being related to a specimen image that has been captured for pathological diagnosis;
a generating unit that generates an evaluator, based on an evaluation recipe that is evaluation setting information indicating a setting related to evaluation of the specimen image, according to the information related to the specimen image, the information having been acquired by the acquiring unit; and
a providing unit that provides, to the terminal apparatus, information related to the evaluator generated by the generating unit, the evaluator being for evaluation of the specimen image that has been corrected according to the evaluator.

23. An information processing system, comprising:
an acquiring unit that acquires information transmitted from a terminal apparatus used by a user according to operation by the user, the information being related to a medical image captured for diagnosis;
a generating unit that generates an evaluator, based on an evaluation recipe that is evaluation setting information indicating a setting related to evaluation of the medical image that has been corrected according to the information related to the medical image, the information having been acquired by the acquiring unit; and
a providing unit that provides, to the terminal apparatus, information related to the evaluator generated by the generating unit, the evaluator being for evaluation of the medical image.

24. An information processing system, comprising:
an acquiring unit that acquires information transmitted from a terminal apparatus used by a user according to operation by the user, the information being related to a medical image captured for diagnosis;
a generating unit that generates an evaluator, based on an evaluation recipe that is evaluation setting information indicating a setting related to evaluation of the medical image, according to the information related to the medical image, the information having been acquired by the acquiring unit; and
a providing unit that provides, to the terminal apparatus, information related to the evaluator generated by the generating unit, the evaluator being for evaluation of the medical image that has been corrected according to the evaluator.
